# EUROPÄISCHE PATENTSCHRIFT

(11) **EP 1 796 627 B1**
(45) Veröffentlichungstag und Bekanntmachung des Hinweises auf die Patenterteilung: **04.03.2009**
(21) Anmeldenummer: 05797305.9
(22) Anmeldetag: 29.09.2005
(51) Int. Cl.: A61Q 5/06, A61K 8/33, A61K 8/49

(54) **MITTEL ZUM FÄRBEN VON KERATINHALTIGEN FASERN**
AGENT FOR COLOURING FIBRES CONTAINING KERATIN
AGENT DE COLORATION POUR DES FIBRES CONTENANT DE LA KERATINE

(30) Priorität: 08.10.2004 DE 102004049363
(43) Veröffentlichungstag der Anmeldung: 20.06.2007
(73) Patentinhaber: Henkel AG & Co. KGaA, 40589 Düsseldorf (DE)
(72) Erfinder: GROSS, Wibke, 40549 Düsseldorf (DE); MAUSBERG, Sandra, 40699 Erkrath (DE); OBERKOBUSCH, Doris, 40591 Düsseldorf (DE); HÖFFKES, Horst, 40595 Düsseldorf (DE)
(86) Internationale Anmeldenummer: PCT/EP2005/010515
(87) Internationale Veröffentlichungsnummer: WO 2006/040015

(56) Entgegenhaltungen:
- EP-A- 1 433 468
- DE-A1- 10 241 076
- H. MÖHRLE, M. WIRTZ: "Zum Identitätsnachweis von Coffein im Arzneibuch; 1. Mitt.: Imidazo[1,5-a]pyrimidiniumsalze" PHARMAZIE, Bd. 54, Nr. 2, 1999, Seiten 115-123, XP001208159
- H. MÖHRLE, M. WIRTZ: "Zum Identitätsnachweis von Coffein im Arzneibuch; 2. Mitt.: Struktur des Farbstoffs aus modifizierter Reaktion" PHARMAZIE, Bd. 54, Nr. 4, 1999, XP001208160

## Beschreibung

Die Erfindung betrifft ein Mittel zum Färben von keratinhaltigen Fasern, insbesondere menschlichen Haaren, das kationische Imidazo[1,5-a]pyrimidinium-Derivate in Kombination mit reaktiven Carbonylverbindungen enthält, die Verwendung dieser Kombination in Mitteln zum Färben von keratinhaltigen Fasern, zur Farbauffrischung bzw. Nuancierung von bereits gefärbten keratinhaltigen Fasern sowie ein Verfahren zum Färben von keratinhaltigen Fasern, insbesondere menschlichen Haaren.

Für das Färben von keratinhaltigen Fasern kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy-oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazol-5-on, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone, m-Aminophenole und substituierte Pyridin-derivate verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 2-Amino-4-(2-hydroxyethylamino)-anisol (Lehmanns Blau), 1-Phenyl-3-methyl-pyrazol-5-on, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin, 5-Methylresorcin, 3-Amino-6-methoxy-2-methylamino-pyridin und 3,5-Diamino-2,6-dimethoxypyridin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe), und Kap. 8, Seiten 264 - 267 (Oxidationsfarbstoffe), sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im allgemeinen unter dem Einfluss von Oxidationsmitteln wie z. B. H₂O₂, was in einigen Fällen Schädigungen der Faser zur Folge haben kann. Problematisch gestaltet sich nach wie vor eine Bereitstellung von Oxidationshaarfärbungen im Rotbereich mit ausreichenden Echtheitseigenschaften, insbesondere in mit sehr guten Wasch- und Reibechtheiten. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Färbemittel, enthaltend CH-acide, kationische Imidazo[1,5-a]pyrimidinium-Derivate gemäß nachstehender Formel I in Kombination mit reaktiven Carbonylverbindungen, sowie die Verwendung dieser Kombination zum Färben von keratinhaltigen Fasern oder zur Farbauffrischung bzw. Nuancierung von bereits gefärbten keratinhaltigen Fasern sind bislang nicht bekannt.

Druckschrift EP-A1-1 433 478 betrifft Haarfärbemittel, die mindestens eine spezielle N-Methyl-aryliminomethyl-chinolinium-Verbindung als direktziehenden Farbstoff enthalten.

In der Druckschrift DE-A1-102 41 076 werden Haarfärbemittel beschrieben, die eine Kombination enthalten aus
(a) mindestens einem speziellen 1,2-Dihydropyrimidiniumderivat mit einer Oxo-, Imino-oder Thioxogruppe in 2-Position und
(b) mindestens einer Verbindung mit reaktiver Carbonylgruppe.

Aufgabe der vorliegenden Erfindung ist es, Färbemittel für keratinhaltige Fasern, insbesondere menschliche Haare, bereitzustellen, die hinsichtlich der Farbtiefe und der Echtheitseigenschaften, wie beispielsweise Licht-, Reib- und Waschechtheit sowie Schweiß- und Kaltwellechtheit, qualitativ den üblichen Oxidationshaarfärbemitteln mindestens gleichwertig sind, ohne jedoch unbedingt auf Oxidationsmittel wie z. B. H₂O₂ angewiesen zu sein. Darüber hinaus dürfen die Färbemittel kein oder lediglich ein sehr geringes Sensibilisierungspotential aufweisen.

Überraschenderweise wurde nun gefunden, daß die in der Formel I dargestellten Verbindungen in Kombination mit Verbindungen enthaltend mindestens eine reaktive Carbonylgruppe, sich auch in Abwesenheit von oxidierenden Agentien hervorragend zum Färben von keratinhaltigen Fasern eignen. Sie ergeben Ausfärbungen mit hervorragender Brillanz und Farbtiefe und führen zu vielfältigen Farbnuancen. Es werden insbesondere Ausfärbungen mit verbesserten Echtheitseigenschaften über einen Nuancenbereich von gelb über orange, braunorange, braun, rot, rotviolett bis hin zu blauviolett und dunkelblau erhalten. Der Einsatz von oxidierenden Agentien soll jedoch nicht prinzipiell ausgeschlossen werden.

Ein erster Gegenstand der Erfindung ist ein Mittel zum Färben von keratinhaltigen Fasern, insbesondere menschlichen Haaren, enthaltend in einem kosmetischen Träger als Komponente A mindestens eine Verbindung gemäß Formel I und/oder deren Enaminform, 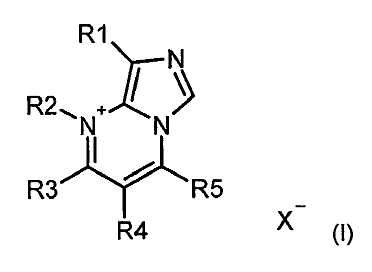 worin
• R¹ steht für ein Wasserstoffatom, ein Halogenatom, eine Carboxylgruppe, eine (C₁-C₆)-Alkoxygruppe, (C₁-C₆)-Alkylgruppe, eine (C₂-C₆)-Alkenylgruppe, eine gegebenenfalls substituierte Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{I}R^{II}N-CO-, worin R^{I} und R^{II} stehen unabhängig voneinander für ein Wasserstoffatom, eine (C₁-C₆)-Alkylgruppe, eine (C₁-C₆)-Alkenylgruppe, eine Arylgruppe oder eine Aryl-C₁-C₆-alkylgruppe, wobei R¹ und R^{II} gemeinsam mit dem Stickstoffatom einen 5-, 6- oder 7-gliedrigen Ring bilden können, und
• R² steht für eine (C₁-C₆)-Alkylgruppe, eine (C₂-C₆)-Alkenylgruppe, eine gegebenenfalls substituierte Arylgruppe, eine Aryl-(C₁-C₆)-alkylgruppe, eine (C₁-C₆)-Hydroxyalkylgruppe, eine (C₂-C₆)-Polyhydroxyalkylgruppe, oder eine Gruppe R^{III}R^{IV}N-(CH₂)ₙ-, worin R^{III} und R^{IV} stehen unabhängig voneinander für ein Wasserstoffatom, eine (C₁-C₆)-Alkylgruppe, eine (C₁-C₆)-Alkenylgruppe oder eine Aryl-C₁-C₆-alkylgruppe, wobei R^{III} und R^{IV} gemeinsam mit dem Stickstoffatom einen 5-, 6- oder 7-gliedrigen Ring bilden können und n steht für eine Zahl 2, 3, 4, 5 oder 6,
• mindestens ein Rest aus R³ oder R⁵ steht für eine Methylgruppe und der andere Rest steht für ein Wasserstoffatom, eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe oder eine (C₂-C₆)-Polyhydroxyalkylgruppe,
• R⁴ steht für ein Wasserstoffatom, eine (C₁-C₆)-Alkylgruppe, eine (C₂-C₆)-Alkenylgruppe, eine (C₁-C₆)-Hydroxyalkylgruppe, eine (C₂-C₆)-Polyhydroxyalkylgruppe oder eine gegebenenfalls substituierte Arylgruppe,
• X⁻ ein physiologisch verträgliches Anion bedeutet
und als Komponente B mindestens eine reaktive Carbonylverbindung.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z. B. Baumwolle, Jute, Sisal, Leinen oder Seide, modifizierter Naturfasern, wie z. B. Regeneratcellulose, Nitro-, Alkyl- oder Hydroxyalkyl-oder Acetylcellulose verwendet werden.

Beispiele für C₁-C₆-Alkylreste sind die Gruppen Methyl, Ethyl, Propyl, Isopropyl, n-Butyl, sec-Butyl und tert.-Butyl, n-Pentyl und n-Hexyl. Propyl, Ethyl und Methyl sind bevorzugte Alkylreste. Beispiele für entsprechende cyclische Alkylgruppen sind Cyclopentyl und Cyclohexyl.
Beispiele für bevorzugte C₂-C₆-Alkenylreste sind Vinyl und Allyl.
Bevorzugte Arylgruppen sind Phenyl, Naphthyl und Biphenyl.
Bevorzugte Aryl-(C₁-C₆)-alkylgruppen sind Benzyl und 2-Phenylethyl.
Weiterhin können als bevorzugte Beispiele für eine C₁-C₆-Hydroxyalkylgruppe eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyethylgruppe genannt werden. Eine 2-Hydroxyethylgruppe ist besonders bevorzugt.
Beispiele für eine C₂-C₆-Polyhydroxyalkylgruppe sind die 2,3-Dihydroxypropylgruppe, 3,4-Dihydroxybutylgruppe und die 2,4-Dihydroxybutylgruppe.

Es ist erfindungsgemäß bevorzugt, wenn der Rest R¹ steht für eine Carbamoylgruppe R^{I}R^{II}N-CO- steht, worin R^{I} und R^{II} unabhängig voneinander ein Wasserstoffatom oder eine (C₁-C₆)-Alkylgruppe, insbesondere eine Methylgruppe, bedeuten.

Es ist bevorzugt, wenn in Formel (I) die Reste R², R³ und R⁵ eine Methylgruppe bedeuten.

Es ist erfindungsgemäß bevorzugt, wenn R⁴ für ein Wasserstoffatom oder eine (C₁-C₆)-Alkylgruppe steht.

Es ist bevorzugt, wenn X⁻ gemäß Formel (I) und nachfolgender Formel (II) ausgewählt wird aus Halogenid (Chlorid, Bromid, lodid), Benzolsulfonat, p-Toluolsulfonat, (C₁- bis C₄)-Alkansulfonat, Trifluormethansulfonat, Acetat, Trifluoracetat, Perchlorat, 0.5 Sulfat, Hydrogensulfat, Tetrafluoroborat, Hexafluorophosphat, Hexafluorozinkat,oder Tetrachlorozinkat. Besonders bevorzugt steht X⁻ für Chlorid, Bromid, Tetrafluoroborat oder Hydrogensulfat.

Vorzugsweise wird die Verbindung gemäß Formel I ausgewählt aus der Gruppe, bestehend aus Salzen mit physiologisch verträglichem Anion X⁻ des 1,2,4-Trimethyl-8-[(methylamino)carbonyl]imidazo[1,5-a]pyrimidiniums (siehe Formel II), des 1,2,3,4-Tetramethyl-8-[(methylamino)carbonyl]imidazo[1,5-a]pyrimidiniums, der 3-Ethyl-1,2,4-trimethyl-8-[(methylamino)carbonyl]imidazo[1,5-a]pyrimidiniums, sowie den Enaminformen der zuvor genannten Salze. Die zur Benennung der erfindungsgemäßen Verbindungen verwendete Nummerierung der Atome des Imidazo[1,5-a]pyrimidinium-Ringgerüsts wird in Formel II illustriert.

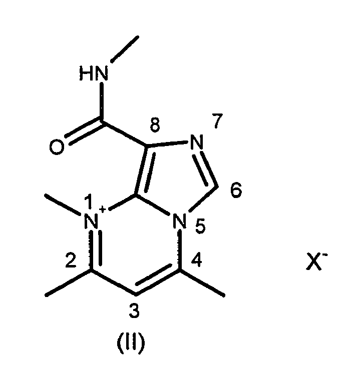

Als CH-acide Verbindungen werden im Allgemeinen solche Verbindungen angesehen, die ein an ein aliphatisches Kohlenstoffatom gebundenes Wasserstoffatom tragen, wobei aufgrund von Elektronen-ziehenden Substituenten eine Aktivierung der entsprechenden Kohlenstoff-Wasserstoff-Bindung bewirkt wird. Die Verbindungen gemäß Formel I sind CH-acide Verbindungen. Mit Hilfe einer Base lässt sich die Enaminform gezielt darstellen. Exemplarisch für Verbindungen gemäß Formel (I) wird die Enaminform nachfolgend als Verbindung der Formel (II) durch die folgenden Formeln (IIa) und (IIb) illustriert.

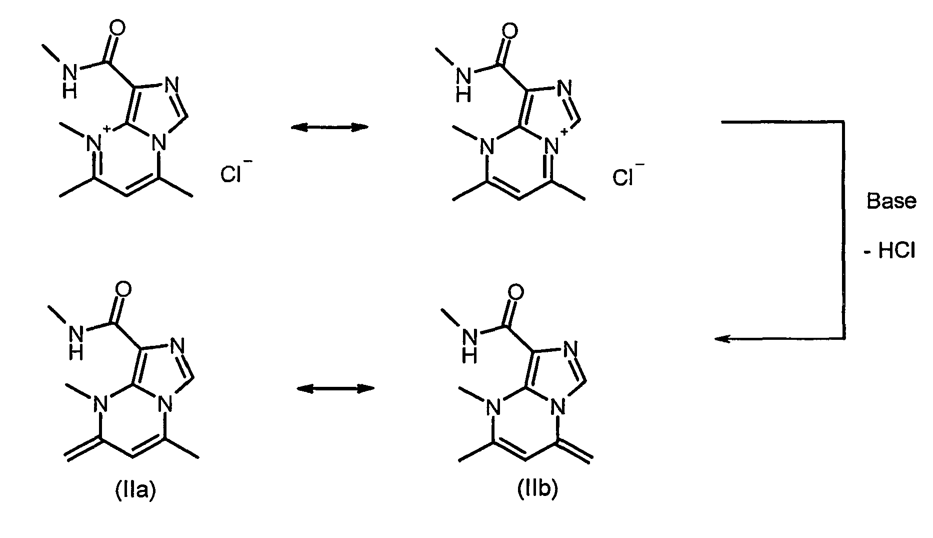

Die CH-aciden Verbindungen der Formel (I) sind allgemein literaturbekannt. Die erfindungsgemäßen Verbindungen der Formel I sind nach bekannten Syntheseverfahren nach H. Möhrle et al., Pharmazie, 1999, 54(2), 115-123 herstellbar.

Reaktive Carbonylverbindungen als Komponente B besitzen im Sinne der Erfindung mindestens eine Carbonylgruppe als reaktive Gruppe, welche mit der CH-aciden Verbindung gemäß Formel I unter Ausbildung einer Kohlenstoff-Kohlenstoff-Bindung reagiert. Bevorzugte reaktive Carbonylverbindungen sind Aldehyde und Ketone, insbesondere aromatische Aldehyde. Ferner sind erfindungsgemäß auch solche Verbindungen als Komponente B verwendbar, in denen die reaktive Carbonylgruppe derart derivatisiert bzw. maskiert ist, daß die Reaktivität des Kohlenstoffatoms der derivatisierten Carbonylgruppe gegenüber den CH-aciden Verbindungen der Formel I stets vorhanden ist. Diese Derivate sind bevorzugt Additionsverbindungen
a) von Aminen und deren Derivate unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente B leitet sich in diesem Fall c) von einem Aldehyd ab)
an das Kohlenstoffatom der Carbonylgruppe der reaktiven Carbonylverbindung.

Bevorzugte reaktive Carbonylverbindungen der Komponente B werden ausgewählt aus der Gruppe, bestehend aus Benzaldehyd und seinen Derivaten, Naphthaldehyd und seinen Derivaten, Zimtaldehyd und seinen Derivaten, 2,3,6,7-Tetrahydro-1H,5H-benzo[ij ]chinolizin-9-carboxaldehyd, 2,3,6,7-Tetrahydro-8-hydroxy-1H,5H-benzo[ij]chinolizin-9-carboxaldehyd, N-Ethylcarbazol-3-aldehyd, 2-Formylmethylen-1,3,3-trimethylindolin (Fischers Aldehyd oder Tribasen Aldehyd), 2-Indolaldehyd, 3-Indolaldehyd, 1-Methylindol-3-aldehyd, 2-Methylindol-3-aldehyd, 2-(1',3',3'-Trimethyl-2-indolinyliden)-acetaldehyd, 1-Methylpyrrol-2-aldehyd, 4-Pyridinaldehyd, 2-Pyridinaldehyd, 3-Pyridinaldehyd, Pyridoxal, Antipyrin-4-aldehyd, Furfural, 5-Nitrofurfural, 2-Thenoyl-trifluor-aceton, Chromon-3-aldehyd, 3-(5'-Nitro-2'-furyl)-acrolein, 3-(2'-Furyl)-acrolein und Imidazol-2-aldehyd, 5-(4-Dimethylaminophenyl)penta-2,4-dienal, 5-(4-Diethylaminophenyl)penta-2,4-dienal, 5-(4-Methoxyphenyl)penta-2,4-dienal, 5-(3,4-Dimethoxyphenyl)penta-2,4-dienal, 5-(2,4-Dimethoxyphenyl)penta-2,4-dienal, 5-(4-Piperidinophenyl)penta-2,4-dienal, 5-(4-Morpholinophenyl)penta-2,4-dienal, 5-(4-Pyrrolidinophenyl)penta-2,4-dienal, 5-(4-Dimethylamino-1-naphthyl)penta-3,5-dienal, 9-Methyl-3-carbazolaldehyd, 9-Ethyl-3-carbazolaldehyd, 3-Acetylcarbazol, 3,6-Diacetyl-9-ethylcarbazol, 3-Acetyl-9-methylcarbazol, 1,4-Dimethyl-3-carbazolaldehyd, 1,4,9-Trimethyl-3-carbazolaldehyd, 6-Nitropiperonal, 2-Nitropiperonal, 5-Nitrovanillin, 2,5-Dinitrosalicylaldehyd, 5-Brom-3-nitrosalicylaldehyd, 3-Nitro-4-formylbenzolsulfonsäure, 4-Formyl-1-methylpyridinium-, 2-Formyl-1-methylpyridinium-, 4-Formyl-1-ethylpyridinium-, 2-Formyl-1-ethylpyridinium-, 4-Formyl-1-benzylpyridinium-, 2-Formyl-1-benzylpyridinium-, 4-Formyl-1,2-dimethylpyridinium-, 4-Formyl-1,3-dimethylpyridinium-, 4-Formyl-1-methylchinolinium-, 2-Formyl-1-methylchinolinium-, 5-Formyl-1-methylchinolinium-, 6-Formyl-1-methylchinolinium-, 7-Formyl-1-methylchinolinium-, 8-Formyl-1-methylchinolinium, 5-Formyl-1-ethylchinolinium-, 6-Formyl-1-ethylchinolinium-, 7-Formyl-1-ethylchinolinium-, 8-Formyl-1-ethylchinolinium, 5-Formyl-1-benzylchinolinium-, 6-Formyl-1-benzylchinolinium-7-Formyl-1-benzylchinolinium-, 8-Formyl-1-benzylchinolinium, 5-Formyl-1-allylchinolinium-, 6-Formyl-1-allylchinolinium-, 7-Formyl-1-allylchinolinium- und 8-Formyl-1-allylchinolinium-benzolsulfonat, -p-toluolsulfonat, -methansulfonat, -perchlorat, -sulfat,-chlorid, -bromid, -iodid, -tetrachlorozinkat, -methylsulfat-, -trifluormethansulfonat,-tetrafluoroborat, Isatin, 1-Methyl-isatin, 1-Allyl-isatin, 1-Hydroxymethyl-isatin, 5-Chlorisatin, 5-Methoxy-isatin, 5-Nitroisatin, 6-Nitro-isatin, 5-Sulfo-isatin, 5-Carboxy-isatin, Chinisatin, 1-Methylchinisatin, sowie beliebigen Gemischen der voranstehenden Verbindungen.

Ganz besonders bevorzugt werden in den erfindungsgemäßen Mitteln Benzaldehyd, Zimtaldehyd und Naphthaldehyd sowie deren Derivate, insbesondere mit einem oder mehreren Hydroxy-, Alkoxy- oder Aminosubstituenten, als reaktive Carbonylverbindung verwendet. Dabei werden wiederum die Verbindungen gemäß Formel (Ca-1) bevorzugt, 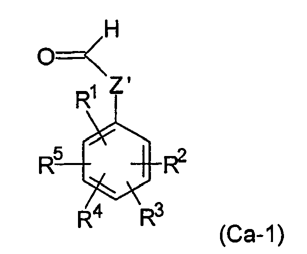 worin
• R¹, R² und R³ stehen unabhängig voneinander für ein Wasserstoffatom, ein Halogenatom, eine C₁-C₆-Alkylgruppe, eine Hydroxygruppe, eine C₁-C₆-Alkoxygruppe, eine C₁-C₆-Dialkylaminogruppe, eine Di(C₂-C₆-hydroxyalkyl)aminogruppe, eine Di(C₁-C₆-alkoxy-C₁-C₆-alkyl)aminoguppe, eine C₁-C₆-Hydroxyalkyloxygruppe, eine Sulfonylgruppe, eine Carboxylgruppe, eine Sulfonsäuregruppe, eine Sulfonamidogruppe, eine Sulfonamidgruppe, eine Carbamoylgruppe, eine C₂-C₆-Acylgruppe, eine Acetylgruppe oder eine Nitrogruppe,
• Z' steht für eine direkte Bindung oder eine Vinylengruppe,
• R⁴ und R⁵ stehen für ein Wasserstoffatom oder bilden gemeinsam, zusammen mit dem Restmolekül einen 5- oder 6-gliederigen aromatischen oder aliphatischen Ring.

Die Derivate der Benzaldehyde, Naphthaldehyde bzw. Zimtaldehyde der reaktiven Carbonylverbindung gemäß Komponente B werden besonders bevorzugt ausgewählt aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzaldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nitrobenzaldehyd, 3-Brom-4-hydroxybenzaldehyd, 4-Hydroxy-3-methylbenzaldehyd, 3,5-Dimethyl-4-hydroxy-benzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethylbenzaldehyd, 4-Hydroxy-2,6-dimethyl-benzaldehyd, 3,5-Diethoxy-4-hydroxybenzaldehyd, 2,6-Diethoxy-4-hydroxy-benzaldehyd, 3-Hydroxy-4-methoxy-benzaldehyd, 2-Hydroxy-4-methoxy-benzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dihydroxy-6-methyl-benzaldehyd, 2,4-Dihydroxy-3-methoxybenzaldehyd, 2,4-Dihydroxy-5-methoxy-benzaldehyd, 2,4-Dihydroxy-6-methoxybenzaldehyd, 2,5-Dihydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxybenzaldehyd, 3,5-Dihydroxybenzaldehyd, 2,3,4-Trihydroxybenzaldehyd, 2,3,5-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiod-benzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1 - naphthaldehyd, 2,4-Dimethoxy-1-naphthaldehyd, 3,4-Dimethoxy-1-naphthaldehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nitrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dimethoxy-2-nitrobenzaldehyd, 6-Nitropiperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-(1-Imidazolyl)-benzaldehyd und Piperonal. Diese Vertreter sind zugleich die besonders bevorzugten zusätzlichen reaktiven Carbonylverbindungen der Komponente B.

In einer zweiten Ausführungsform kann es zur Erweiterung des Farbspektrums vorteilhaft sein, den erfindungsgemäßen Mitteln neben mindestens einer Verbindung gemäß Formel (I) als Komponente A und mindestens einer Verbindung der Komponente B mindestens eine weitere Verbindung als Komponente C zuzusetzen. Die Verbindung der Komponente C wird bevorzugt ausgewählt aus CH-aciden Verbindungen, welche von Verbindungen der Formel (I) verschieden sind.

Die zusätzlichen CH-aciden Verbindungen der Komponente C sind bevorzugt ausgewählt aus der Gruppe bestehend aus mit physiologisch verträglichen Anionen, insbesondere p-Toluolsulfonaten, Methansulfonaten, Hydrogensulfaten, Tetrafluoroboraten und Halogeniden, wie den Chloriden, Bromiden und Iodiden, gebildeten Salzen des 1,4-Dimethylchinoliniums, 1-Ethyl-4-methyl-chinoliniums, 1-Ethyl-2-methylchinoliniums, 1,2,3,3-Tetramethyl-3H-indoliums, 2,3-Dimethyl-benzothiazoliums, 2,3-Dimethyl-naphtho[1,2-d]thiazoliums, 3-Ethyl-2-methyl-naphtho[1,2-d]thiazoliums, 3-Ethyl-2-methyl-benzoxazoliums, 1,2,3-Trimethylchinoxaliniums, 3-Ethyl-2-methyl-benzothiazoliums, 1,2-Dihydro-1,3,4,6-tetramethyl-2-oxo-pyrimidiniums, 1,2-Dihydro-1,3,4-trimethyl-2-oxo-pyrimidiniums, 1,2-Dihydro-4,6-dimethyl-1,3-dipropyl-2-oxo-pyrimidiniums, 1,2-Dihydro-1,3,4,6-tetramethyl-2-thioxo-pyrimidiniums, 1,2-Dihydro-1,3,4,5,6-pentamethyl-2-oxo-pyrimidiniums, 2,5-Dimethyl-3-(2-propenyl)-1,3,4-thiadiazoliums, 3-Ethyl-2,5-dimethyl-1,3,4-thiadiazoliums, 1,2-Dimethylchinoliniums und 1,3,3-Trimethyl-2-methylenindolin (Fischersche Base), Oxindol, 3-Methyl-1-phenyl-pyrazolin-5-on, Indan-1,2-dion, Indan-1,3-dion, Indan-1-on, 2-Amino-4-imino-1,3-thiazolin-hydrochlorid, Benzoylacetonitril, 3-Dicyanmethylenindan-1-on, 2-(2-Furanoyl)acetonitril, 2-(2-Theonyl)acetonitril, 2-(Cyanmethyl)benzimidazol, 2-(Cyanmethyl)-benzothiazol und 2-(2,5-Dimethyl-3-furanoyl)acetonitril.

In einer dritten Ausführungsform enthält das Färbemittel zusätzlich mindestens ein Reaktionsprodukt (im folgenden als Reaktionsprodukt RP bezeichnet) aus einer Verbindung der Formel I und einer Verbindung der Komponente B als direktziehenden Farbstoff. Derartige Reaktionsprodukte RP können z. B. durch Erwärmen der beiden Reaktionspartner in wässrigem neutralen bis schwach alkalischen Milieu erhalten werden, wobei die Reaktionsprodukte RP entweder als Feststoff aus der Lösung ausfallen oder durch Eindampfen der Lösung daraus isoliert werden. Ferner besteht die Möglichkeit, die Reaktionsprodukte gemäß Literatur H. Möhrle et al, Pharmazie, 1999, 54(4), 269-279 darzustellen.

Zur Synthese der Reaktionsprodukte RP können Molverhältnisse der Komponente B zu der Verbindung gemäß Formel I von etwa 1:1 1 bis etwa 2:1 sinnvoll sein.

Die voranstehend genannten Verbindungen mit der Formel I, die Verbindungen der Komponente B, Komponente C sowie die Reaktionsprodukte RP werden jeweils vorzugsweise in einer Menge von 0,03 bis 65 mmol, insbesondere von 1 bis 40 mmol, bezogen auf 100 g des gesamten Färbemittels, verwendet.

Zusätzlich können die erfindungsgemäßen Mittel mindestens eine Entwicklerkomponente und gegebenenfalls mindestens eine Kupplerkomponente als Oxidationsfarbstoffvorprodukte enthalten.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 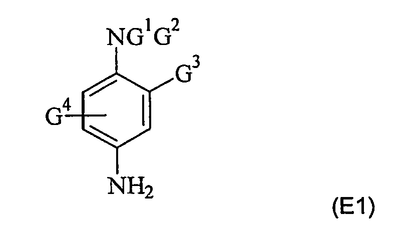 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, Iod-oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, CI-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin, N-(4-Amino-3-methylphenyl)-N-[3-(1H-imidazol-1-yl)propyl]amin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 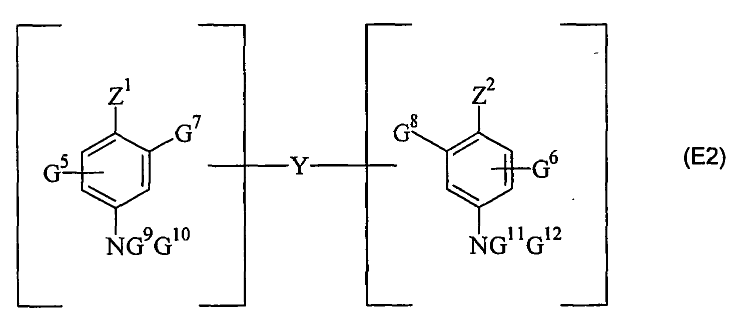 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄- Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest,
mit der Maßgabe, dass die Verbindungen der Formel (E2) nur eine Verbrückung Y pro Molekül enthalten.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4-aminophenyl)-1,4-diazacycloheptan und 1,10-Bis-(2,5-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 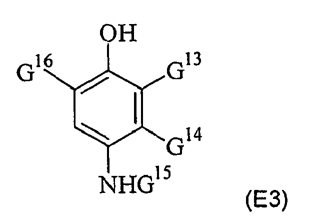 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest, einen Hydroxy-(C₁-bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazolopyrimidin-Derivate sind insbesondere die Derivate des Pyrazolo[1,5-a]pyrimidin der folgenden Formel (E4) und dessen tautomeren Formen, sofern ein tautomeres Gleichgewicht besteht: 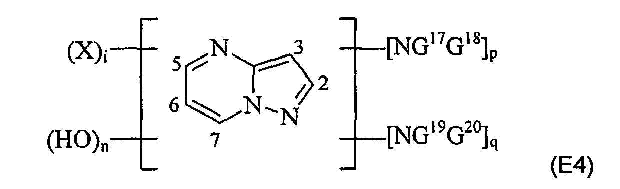 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂-bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁-bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁-bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁-bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁-bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
   mit der Maßgabe, dass
- die Summe aus p + q ungleich 0 ist,
- wenn p + q gleich 2 ist, n den Wert 0 hat, und die Gruppen NG¹⁷G¹⁸ und _{NG}¹⁹_{G}²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazolo[1,5-a]pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird: 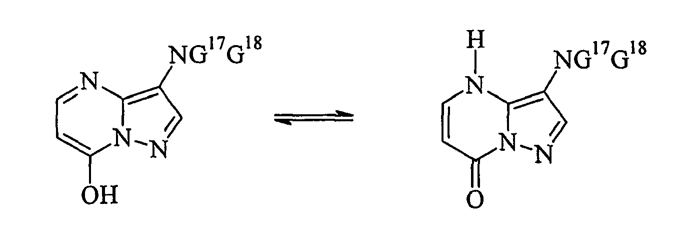 Unter den Pyrazolo[1,5-a]pyrimidinen der obenstehenden Formel (E4) kann man insbesondere nennen:
- Pyrazolo[1,5-a]pyrimidin-3,7-diamin;
- 2,5-Dimethylpyrazolo[1,5-a]-pyrimidin-3,7-diamin;
- Pyrazolo[1,5-a]pyrimidin-3,5-diamin;
- 2,7-Dimethyl-pyrazolo[1,5-a]pyrimidin-3,5-diamin;
- 3-Aminopyrazolo[1,5-a]pyrimidin-7-ol;
- 3-Aminopyrazolo[1,5-a]pyrimidin-5-ol;
- 2-(3-Aminopyrazolo[1,5-a]pyrimidin-7-ylamino)-ethanol;
- 2-(7 -Aminopyrazolo[1,5-a]pyrimidin-3-ylamino)-ethanol;
- 2-[(3-Aminopyrazolo[1,5-a]pyrimidin-7-yl)-(2-hydroxy-ethyl)-amino]-ethanol;
- 2-[(7-Aminopyrazolo[1,5-a]pyrimidin-3-yl)-(2-hydroxy-ethyl)-amino]-ethanol;
- 5,6-Dimethylpyrazolo[1,5-a]pyrimidin-3,7-diamin;
- 2,6-Dimethylpyrazolo[1,5-a]pyrimidin-3,7-diamin;
- 3-Amino-7-dimethylamino-2,5-dimethylpyrazolo[1,5-a]pyrimidin;
sowie ihre physiologisch verträglichen Salze und ihre tautomeren Formen, wenn ein tautomeres Gleichgewicht vorhanden ist.

Die Pyrazolo1,5-a]pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-[3-Morpholin-4-ylphenyl)amino]ethanol, 3-Amino-4-(2-methoxyethoxy)-5-methylphenylamin und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Aminobenzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol
sowie deren physiologisch verträglichen Salze.

Erfindungsgemäß besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Zusätzlich können im Rahmen einer fünften Ausführungsform als Vorstufen naturanaloger Farbstoffe bevorzugt solche Indole und Indoline in den erfindungsgemäßen Mitteln eingesetzt werden, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel IIIa, 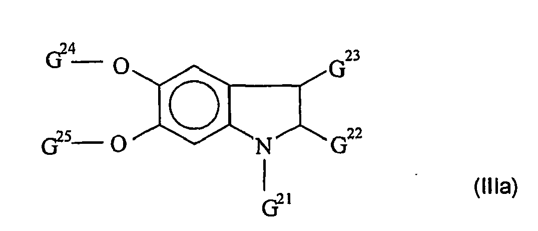 in der unabhängig voneinander
- G²¹ steht für Wasserstoff, eine C₁-C₄-Alkylgruppe oder eine C₁-C₄-Hydroxy-alkylgruppe,
- G²² steht für Wasserstoff oder eine -COOH-Gruppe, wobei die -COOH-Gruppe auch als Salz mit einem physiologisch verträglichen Kation vorliegen kann,
- G²³ steht für Wasserstoff oder eine C₁-C₄-Alkylgruppe,
- G²⁴ steht für Wasserstoff, eine C₁-C₄-Alkylgruppe oder eine Gruppe -CO-G²⁶, in der G²⁶ steht für eine C₁-C₄-Alkylgruppe, und
- G²⁵ steht für eine der unter G²⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbonsäure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel IIIb, 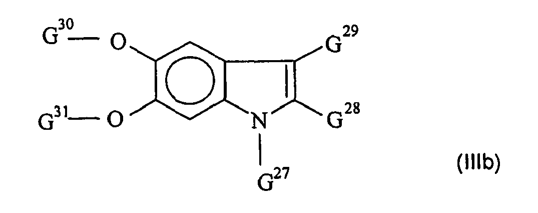 in der unabhängig voneinander
- G²⁷ steht für Wasserstoff, eine C₁-C₄-Alkylgruppe oder eine C₁-C₄-Hydroxyalkylgruppe,
- G²⁸ steht für Wasserstoff oder eine -COOH-Gruppe, wobei die -COOH-Gruppe auch als Salz mit einem physiologisch verträglichen Kation vorliegen kann,
- G²⁹ steht für Wasserstoff oder eine C₁-C₄-Alkylgruppe,
- G³⁰ steht für Wasserstoff, eine C₁-C₄-Alkylgruppe oder eine Gruppe -CO-G³², in der G³² steht für eine C₁-C₄-Alkylgruppe, und
- G³¹ steht für eine der unter G³⁰ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

Auf die Anwesenheit von Oxidationsmitteln, z. B. H₂O₂, kann, insbesondere wenn das erfindungsgemäße Mittel keine Oxidationsfarbstoffvorprodukte enthält, verzichtet werden. Wenn das erfindungsgemäße Mittel luftoxidable Oxidationsfarbstoffvorprodukte oder Indol bzw. Indolinderivate enthält, kann in einem solchen Fall ohne Probleme auf Oxidationsmittel verzichtet werden. Es kann jedoch u. U. wünschenswert sein, den erfindungsgemäßen Mitteln zur Erzielung der Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂. Auch Gemische von mehreren Oxidationsmitteln, wie beispielsweise eine Kombination aus Wasserstoffperoxid und Peroxodisulfaten der Alkali- und Erdalkalimetalle oder aus lodidionenquellen, wie z.B. Alkalimetalliodiden und Wasserstoffperoxid oder den vorgenannten Peroxodisulfaten, können verwendet werden. Das Oxidationsmittel bzw. die Oxidationsmittelkombination können erfindungsgemäß in Verbindung mit Oxidationskatalysatoren in dem Haarfärbemittel zur Anwendung kommen. Oxidationskatalysatoren sind beispielsweise Metallsalze, Metallchelat-Komplexe oder Metalloxide, die einen leichten Wechsel zwischen zwei Oxidationsstufen der Metallionen ermöglichen. Beispiele sind Salze, Chelatkomplexe oder Oxide von Eisen, Ruthenium, Mangan und Kupfer. Weitere mögliche Oxidationskatalysatoren stellen Enzyme dar. Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder *in situ* geringe Mengen Wasserstoffperoxid erzeugen und auf diese Weise die Oxidation der Farbstoffvorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

In einer sechsten Ausführungsform enthalten die erfindungsgemäßen Färbemittel zur weiteren Modifizierung der Farbnuancen neben den erfindungsgemäß enthaltenen Verbindungen zusätzlich übliche direktziehende Farbstoffe, wie Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel bevorzugt einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

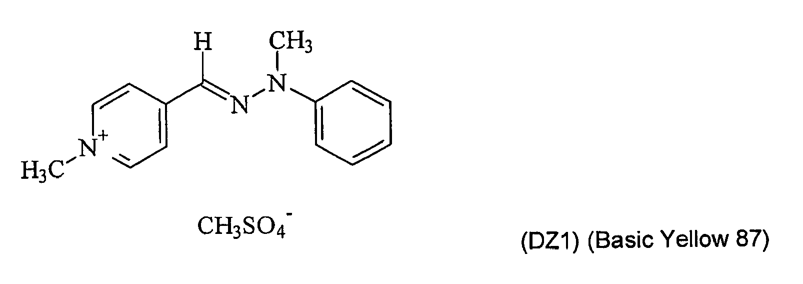

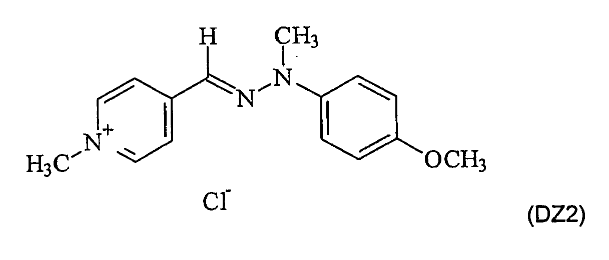

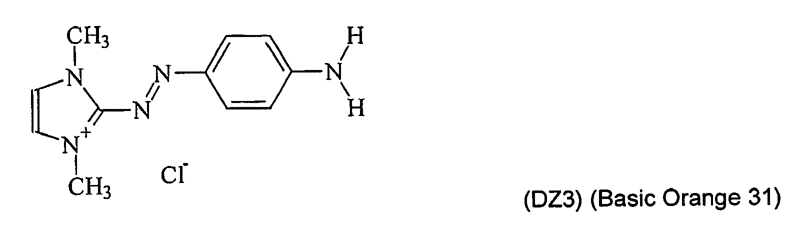

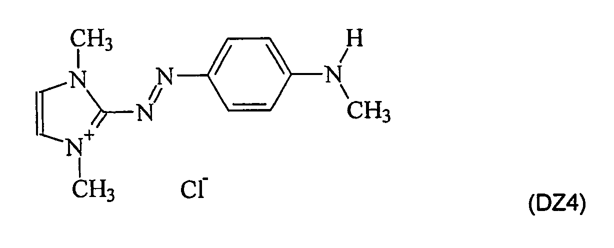

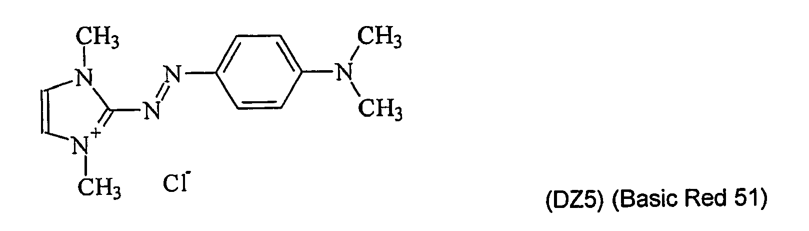

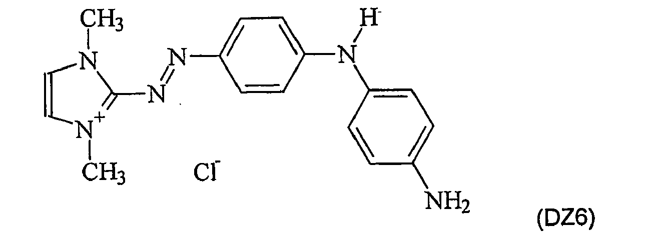

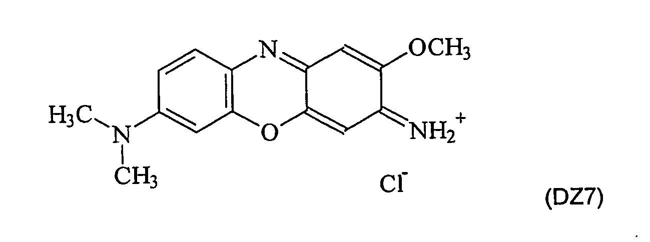

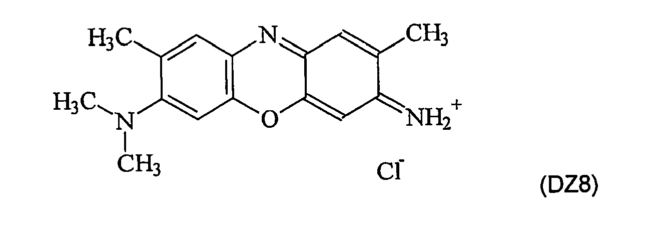

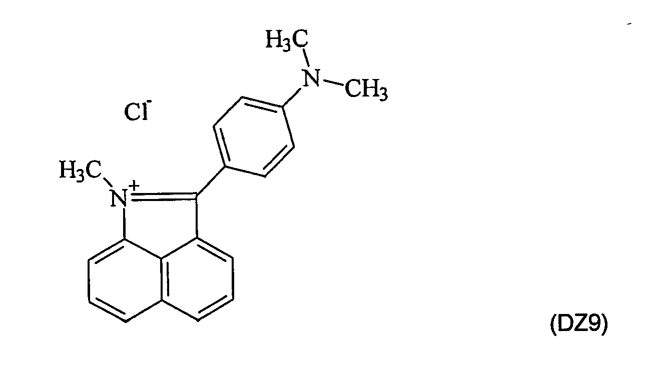

Die Verbindungen der Formeln (DZ1), (DZ3) und (DZ5) sind ganz besonders bevorzugte kationische direktziehende Farbstoffe der Gruppe (c). Die kationischen direktziehenden Farbstoffe, die unter dem Warenzeichen Arianor^{®} vertrieben werden, sind erfindungsgemäß besonders bevorzugte direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, daß die fakultativ enthaltenen direktziehenden Farbstoffe jeweils einheitliche Verbindungen darstellen. Vielmehr können in den erfindungsgemäßen Färbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Zur Erlangung weiterer und intensiverer Ausfärbungen können die erfindungsgemäßen Mittel zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Die erfindungsgemäßen Mittel können einen pH-Wert von pH 4 bis 12, bevorzugt von pH 5 bis 10 besitzen.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die Färbemittel üblicherweise in einen wasserhaltigen kosmetischen Träger eingearbeitet werden. Geeignete wasserhaltige kosmetische Träger sind z. B. Cremes, Emulsionen, Gele oder auch tensidhaltige schäumende Lösungen wie z. B. Shampoos oder andere Zubereitungen, die für die Anwendung auf den keratinhaltigen Fasern geeignet sind. Falls erforderlich ist es auch möglich, die Färbemittel in wasserfreie Träger einzuarbeiten. Beispiele weiterer geeigneter und erfindungsgemäß bevorzugter Inhaltsstoffe sind nachstehend angegeben.

In vielen Fällen enthalten die Färbemittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2 bis 15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungsprodukte von Ethylenoxid an Sorbitanfettsäureester
- Anlagerungsprodukte von Ethylenoxid an Fettsäurealkanolamide.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Weitere Wirk-, Hilfs- und Zusatzstoffe sind beispielsweise
- nichtionische Polymere wie beispielsweise VinylpyrrolidonNinylacrylat-Copolymere, Polyvinylpyrrolidon und VinylpyrrolidonNinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methylmethacrylat/tert.-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und AcrylsäurelEthylacrylat/N-tert.-Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z. B. Polyvinylalkohol,
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrin,
- Lösungsvermittler wie Ethanol, Isopropanol, Ethylenglykol, Propylenglykol, Glycerin und Diethylenglykol,
- Antischuppenwirkstoffe wie Piroctone Olamine und Zink Omadine,
- weitere Substanzen zur Einstellung des pH-Wertes, wie Ammoniak, Monoethanolamin, basische Aminosäuren und Citronensäure
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Die Bestandteile des wasserhaltigen Trägers werden zur Herstellung der erfindungsgemäßen Färbemittel in für diesen Zweck üblichen Mengen eingesetzt; z. B. werden Emulgiermittel in Konzentrationen von 0,5 bis 30 Gew.-% und Verdickungsmittel in Konzentrationen von 0,1 bis 25 Gew.-% des gesamten Färbemittels eingesetzt.

Für das Färbeergebnis kann es vorteilhaft sein, den Färbemitteln Ammonium- oder Metallsalze zuzugeben. Geeignete Metallsalze sind z. B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Alkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calciumbromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65 mmol, insbesondere von 1 bis 40 mmol, bezogen auf 100 g des gesamten Färbemittels, enthalten.

Der pH-Wert der gebrauchsfertigen Färbezubereitungen liegt üblicherweise zwischen 2 und 11, vorzugsweise zwischen 5 und 10.

Ein zweiter Gegenstand der vorliegenden Erfindung betrifft die Verwendung von mindestens einer Verbindung gemäß Formel I und/oder deren Enaminform, 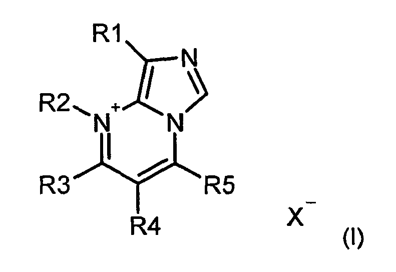 wobei R¹, R², R³, R⁴, R⁵ und X⁻ wie im ersten Erfindungsgegenstand beschrieben definiert sind, zusammen mit mindestens einer reaktiven Carbonylverbindung als Komponente B als färbende Komponente in Haarfärbemitteln.

In einer bevorzugten Ausführungsform verwendet man diejenigen Verbindungen gemäß Formel I als färbende Komponente in Haarfärbemitteln, welche aus den im ersten Erfindungsgegenstand benannten bevorzugten und besonders bevorzugten Vertretern ausgewählt werden.

Darüber hinaus kann es bevorzugt sein, mindestens ein Reaktionsprodukt RP aus einer Verbindung gemäß Formel I und einem Vertreter der Komponente B als färbende Komponenten in Haarfärbemitteln zu verwenden.

Ein dritter Gegenstand der vorliegenden Erfindung betrifft ein Verfahren zum Färben von keratinhaltigen Fasern, insbesondere menschlichen Haaren, worin ein Färbemittel, enthaltend mindestens eine Verbindung gemäß Formel I und/oder deren Enaminform, 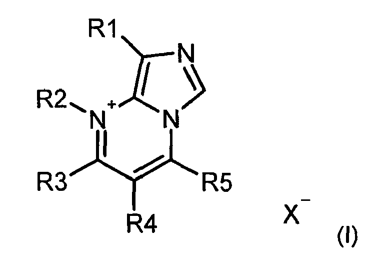 wobei R¹, R², R³, R⁴, R⁵ und X⁻ wie im ersten Erfindungsgegenstand beschrieben definiert sind, zusammen mit mindestens einer reaktiven Carbonylverbindung als Komponente B auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird. Während der Einwirkzeit des Mittels auf der Faser kann es vorteilhaft sein, den Färbevorgang durch Wärmezufuhr zu unterstützen. Die Wärmezufuhr kann durch eine externe Wärmequelle, wie z.B. warme Luft eines Warmluftgebläses, als auch, insbesondere bei einer Haarfärbung am lebenden Probanden, durch die Körpertemperatur des Probanden erfolgen. Bei letzterer Möglichkeit wird üblicherweise die zu färbende Partie mit einer Haube abgedeckt.

Dabei können die Verbindungen gemäß Formel I und die Verbindungen der Komponente B, insbesondere deren vorstehend benannte bevorzugte und besonders bevorzugte Vertreter, als farbgebende Komponenten entweder gleichzeitig auf das Haar aufgebracht werden oder aber auch nacheinander, d. h. in einem mehrstufigen Verfahren, wobei es unerheblich ist, welche der Komponenten zuerst aufgetragen wird. Die fakultativ enthaltenen Ammonium- oder Metallsalze können dabei den Verbindungen mit der Formel I oder den Verbindungen der Komponente B zugesetzt werden. Zwischen dem Auftragen der einzelnen Komponenten können bis zu 30 Minuten Zeitabstand liegen. Auch eine Vorbehandlung der Fasern mit der Salzlösung ist möglich.

Vor der Anwendung des erfindungsgemäßen Mittels in dem erfindungsgemäßen Verfahren kann es wünschenswert sein, die zu färbende keratinhaltige Faser einer Vorbehandlung zu unterziehen. Die zeitliche Abfolge des dazu erforderlichen Vorbehandlungsschritts und der Anwendung des erfindungsgemäßen Mittels muß nicht unmittelbar nacheinander sein, sondern es kann zwischen dem Vorbehandlungsschritt und der Anwendung des erfindungsgemäßen Mittels ein Zeitraum von bis maximal zwei Wochen liegen. Dazu eignen sich mehrere Vorbehandlungsmethoden. Bevorzugt wird die Faser
- V1: vor der Anwendung des erfindungsgemäßen Mittels einer Blondierung oder
- V2: vor der Anwendung des erfindungsgemäßen Mittels einer oxidativen Färbung
unterzogen.

Im Rahmen der Vorbehandlung V1 wird die keratinhaltige Faser mit einem Blondiermittel behandelt. Das Blondiermittel enthält neben einem Oxidationsmittel, wie üblicherweise Wasserstoffperoxid, bevorzugt mindestens ein als Oxidations- und Bleichverstärker wirksames anorganisches Persalz, wie z.B. ein Peroxodisulfat von Natrium, Kalium oder Ammonium. Färbungen gemäß des erfindungsgemäßen Verfahrens erhalten durch die Vorbehandlung V1 eine besondere Brillanz und Farbtiefe.

Im Rahmen der Vorbehandlung V2 wird ein Mittel enthaltend vorgenannte Oxidationsfarbstoffvorprodukte als Entwickler- und gegebenenfalls Kupplerkomponenten sowie gegebenenfalls vorgenannte Derivate des Indols bzw. Indolins auf die Faser aufgetragen und nach einer Einwirkzeit gegebenenfalls unter Zusatz von vorgenannten geeigneten Oxidationsmitteln auf dem Haar für 5-45 Minuten auf der Keratinfaser belassen. Danach wird das Haar gespült. Durch die anschließende Anwendung des erfindungsgemäßen Mittels kann vorhandenen Oxidationsfärbungen einen neue Farbnuance verliehen werden. Wählt man die Farbnuance des erfindungsgemäßen Mittels in der gleichen Farbnuance der oxidativen Färbung aus, so kann die Färbung vorhandener Oxidationsfärbungen nach dem erfindungsgemäßen Verfahren aufgefrischt werden. Es zeigt sich, daß die Farbauffrischung oder Nuancierung gemäß des erfindungsgemäßen Verfahrens einer Farbauffrischung bzw. Nuancierung allein mit herkömmlichen direktziehenden Farbstoffen in der Farbbrillanz und Farbtiefe überlegen ist.

Enthält das Haarfärbemittel neben den Verbindungen gemäß Formel I und den Verbindungen der Komponente B zusätzlich als Oxidationsmittel Wasserstoffperoxid oder ein wasserstoffperoxidhaltiges Oxidationsmittelgemisch, so liegt der pH-Wert des wasserstoffperoxidhaltigen Haarfärbemittels vorzugsweise in einem pH-Bereich von pH 7 bis pH 11, besonders bevorzugt pH 8 bis pH 10. Das Oxidationsmittel kann unmittelbar vor der Anwendung mit dem Haarfärbemittel gemischt und die Mischung auf das Haar aufgebracht werden. Werden die Verbindungen der Formel I und die Komponente B in einem zweistufigen Verfahren auf das Haar appliziert, ist das Oxidationsmittel in einer der beiden Verfahrensstufen zusammen mit der entsprechenden farbgebenden Komponente anzuwenden. Zu diesem Zweck kann es bevorzugt sein, das Oxidationsmittel mit einer der farbgebenden Komponenten in einem Container zu konfektionieren.

Die Verbindungen gemäß Formel I und die Verbindungen der Komponente B können entweder in getrennten Containern oder gemeinsam in einem Container gelagert werden, entweder in einer flüssigen bis pastösen Zubereitung (wässrig oder wasserfrei) oder als Feststoff, beispielsweise als trockenes Pulver. Werden die Komponenten gemeinsam in einer flüssigen Zubereitung gelagert, so sollte diese zur Verminderung einer Reaktion der Komponenten weitgehend wasserfrei sein und einen sauren pH-Wert besitzen. Werden die Komponenten gemeinsam gelagert, so ist es bevorzugt, diese als Feststoff, insbesondere in Form eines bevorzugt mehrschichtigen Formkörpers, z.B. als Tablette zu konfektionieren. Im Falle der mehrschichtigen Formkörper wird die Komponente A in eine Schicht und die Komponente B in eine andere Schicht eingearbeitet, wobei zwischen diesen Schichten vorzugsweise eine weitere Schicht als Trennschicht liegt. Die Trennschicht ist frei von Verbindungen der Komponenten A und B.

Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

Ein vierter Gegenstand der Erfindung ist die Verwendung von mindestens einer Verbindung gemäß Formel I und/oder deren Enaminform, 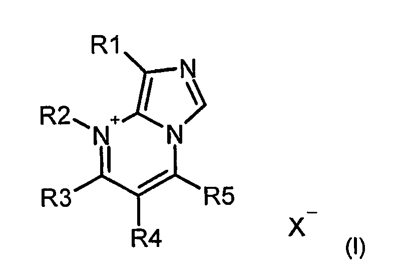 wobei R¹, R², R³, R⁴, R⁵ und X⁻ wie im ersten Erfindungsgegenstand beschrieben definiert sind, zusammen mit mindestens einer reaktiven Carbonylverbindung als Komponente B zur Nuancierung von Oxidationsfärbungen von keratinhaltigen Fasern, insbesondere menschlichen Haaren. Bei der Verwendung ist es unerheblich, ob die Nuancierung gleichzeitig während der oxidativen Färbung erfolgt, oder die oxidative Färbung zeitlich vor der Nuancierung liegt.

Ein fünfter Gegenstand der Erfindung ist die Verwendung von mindestens einer Verbindung gemäß Formel I und/oder deren Enaminform, 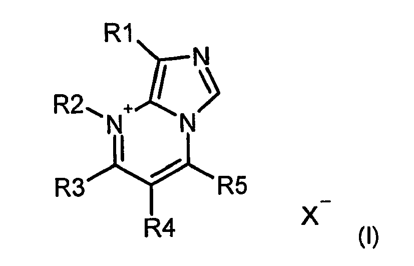 wobei R¹, R², R³, R⁴, R⁵ und X⁻ wie im ersten Erfindungsgegenstand beschrieben definiert sind, zusammen mit mindestens einer reaktiven Carbonylverbindung als Komponente B zur Farbauffrischung von mit oxidativen Färbemitteln gefärbten keratinhaltigen Fasern.

Die Färbungen keratinhaltiger Fasern sind bekanntermaßen Umwelteinflüssen, wie beispielsweise Licht, Reibung oder Waschungen, ausgesetzt und können dadurch an Brillanz und Farbtiefe verlieren. Schlimmstenfalls stellt sich gegebenenfalls eine Nuancenverschiebung der Färbung ein. Solche gealterten Färbungen keratinhaltiger Fasern können, wenn der Anwender es wünscht, durch eine Farbauffrischung wieder annähernd in den farblichen Zustand versetzt werden, wie er sich unmittelbar nach der ursprünglichen Färbung präsentierte. Es ist erfindungsgemäß, für eine solche Farbauffrischung eine Kombination aus mindestens einer Verbindung mit der Formel I und mindestens einer Verbindung der Komponente B zu verwenden.

### Beispiele

### 1.0 Synthese von 1,2,4-Trimethyl-8-[(methylamino)carbonyl]imidazo[1,5-a]pyrimidinium-chlorid

### 1. Stufe:

### Synthese von N-Methyl-4-(methylamino)-1H-imidazol-5-carboxamid (Theophyllidin), Hydrochlorid

Es wurden 50,0 g (0,275 mol) Theophyllin in 250 ml 50%iger Kaliumhydroxydlösung für ca. 6 Stunden erhitzt. Die Reaktionslösung wurde heiß filtriert. Beim Abkühlen des Filtrats bildete sich ein Niederschlag, welcher abfiltriert wurde. Der Feststoff wurde in Wasser gelöst, anschließend wurde mit konzentrierter Salzsäure bis auf einen pH-Wert von 1 angesäuert. Hierbei bildete sich erneut ein Niederschlag, welcher nach Filtration mit Wasser gewaschen wurde. Zur Aufreinigung kann aus Methanol umkristallisiert werden.
Ausbeute: 48,0 g (92 %)
Schmelzpunkt.: 204 - 206 °C

### 2. Stufe:

### Synthese von 1,2,4-Trimethyl-8-[(methylamino)carbonyl]imidazo[1,5-a]pyrimidinium-chlorid (siehe untenstehende Formel II)

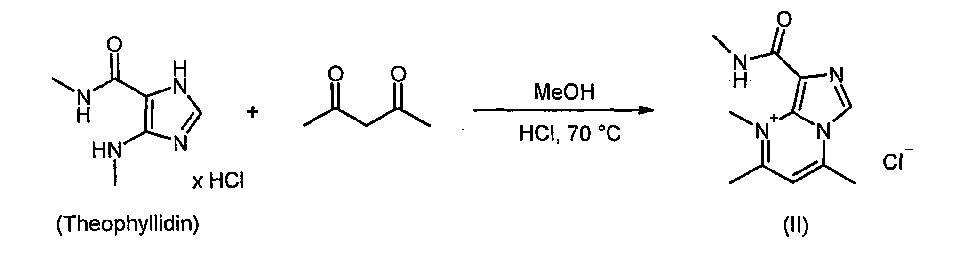

In 1400 ml Methanol wurden 27,0 g (0,142 mol) Theophyllidin (Hydrochlorid) aus Stufe 1 zusammen mit 28,7 g (0,284 mol) Acetylaceton und 20 g konzentrierter Salzsäure für 5 Stunden unter Rückfluß erhitzt. Anschließend wurde das Lösungsmittel am Rotationsverdampfer entfernt und der gelbe Rückstand durch Umkristallisation mittels Ethanol / Diethylether aufgereinigt.
Ausbeute: 33,5 g (93 %)
Smp.: 180 °C

### 2.0 Herstellung der Färbemittel

| Wässrige Gelformulierung für Komponente A | Gel 1: |
|---|---|
| CH-acide Verbindung (Komponente A) | 10 mmol |
| Natrosol HR 250 | 2g |
| Wasser, vollentsalzt | ad 100 g |

Die CH-acide Verbindung (Komponente A) wurde zunächst unter Rühren in wenig Wasser gelöst, dann wurde mit Wasser auf 98 g aufgefüllt. Unter Rühren wurde das Natrosol zugegeben und das Ende des Quellvorgangs abgewartet.

| Wässrige Gelformulierung für Komponente B | Gel 2: |
|---|---|
| Carbonylverbindung (Komponente B) | 10 mmol |
| Natrosol HR 250 | 2g |
| NaOH (50%ige wässrige Lösung) | evtl. einige Tropfen |
| Wasser, vollentsalzt | ad 100 g |

Die Carbonylverbindung (Komponente B) wurde in wenig Wasser gelöst bzw. suspendiert. Zur Erhöhung der Löslichkeit wurde bei Bedarf mit einigen Tropfen 50%iger Natronlauge alkalisiert. Anschließend wurde mit Wasser auf 98 g aufgefüllt und bis zur vollständigen Lösung der Carbonylverbindung gerührt (teilweise unter gelindem Erwärmen auf ca. 40 °C). Anschließend wurde unter Rühren das Natrosol hinzugegeben und der Quellvorgang abgewartet.

### 3.0 Ausfärbungen

Es wurden wässrige Gelformulierungen aus Punkt 2.0 (Gel 1 und Gel 2) mit den Farbstoffvorprodukt Kombinationen der Tabelle 1 hergestellt. Die Gele wurden im Gewichtsverhältnis 1 : 1 vermischt, dann wurde der pH-Wert mit Ammoniak bzw. Weinsäure auf einen Wert von 6 oder 8 (siehe Tabelle 1) eingestellt. Es wurden die Farbstoffvorprodukte in den Kombinationen verwendet, die in Tabelle 1 aufgeführt sind.

Das erhaltene gebrauchsfertige Haarfärbemittel wurde auf einer Strähne zu 90% ergrauten, nicht vorbehandelten Menschenhaares aufgebracht (Flotten-Gewichtsverhältnis: Gelmischung zu Haare = 2 zu 1) und mit einer Applicette gleichmäßig verteilt. Nach einer Einwirkzeit von 30 Minuten bei 32 °C wurde die Strähne mit lauwarmem Wasser ausgespült und danach im warmen Luftstrom (30°C bis 40°C) getrocknet. Die Färbungen wurden unter einer Tageslichtlampe beurteilt. Die Färbeergebnisse sind der Tabelle 1 zu entnehmen.

### Verbindungen der Komponente A (Tabelle 1):

- A1: 1,2,4-Trimethyl-8-[(methylamino)carbonyl]imidazo[1,5-a]pyrimidinium-Chlorid

### Verbindungen der Komponente B (Tabelle 1):

- B1: 2,4-Dihydroxybenzaldehyd
- B2: Vanillin (4-Hydroxy-3-methoxybenzaldehyd)
- B3: 3,5-Dimethoxy-4-hydroxybenzaldehyd
- B4: 4-Hydroxy-1-naphthaldehyd
- B5: 4-Hydroxy-2-methoxybenzaldehyd
- B6: 3,4-Dihydroxybenzaldehyd
- B7: 4-Hydroxybenzaldehyd

**Tabelle 1:**

| Komponente A (Gel 1) | Komponente B (Gel 2) | pH-Wert | Farbton |
|---|---|---|---|
| A1 | B1 | 6 | intensiv rotviolett |
| A1 | B1 | 8 | hell rotviolett |
| A1 | B2 | 6 | intensiv blau |
| A1 | B2 | 8 | pastellblau |
| A1 | B3 | 6 | intensiv dunkelblau |
| A1 | B3 | 8 | hellblau / türkis |
| A1 | B4 | 6 | dunkel grünblau |
| A1 | B4 | 8 | türkis |
| A1 | B5 | 6 | leuchtend violett |
| A1 | B5 | 8 | blass violett |
| A1 | B6 | 6 | intensiv dunkelblau |
| A1 | B6 | 8 | mittelblau |
| A1 | B7 | 6 | intensiv braunrot |
| A1 | B7 | 8 | blass braunrot |

## Patentansprüche

1. Mittel zum Färben von keratinhaltigen Fasern, insbesondere menschlichen Haaren, enthaltend in einem kosmetischen Träger als Komponente A mindestens eine Verbindung gemäß Formel I und/oder deren Enaminform, 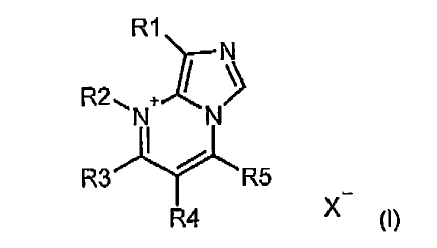 worin
• R¹ steht für ein Wasserstoffatom, ein Halogenatom, eine Carboxylgruppe, eine (C₁-C₆)-Alkoxygruppe, (C₁-C₆)-Alkylgruppe, eine (C₂-C₆)-Alkenylgruppe, eine gegebenenfalls substitulerte Arylgruppe, eine Aryl-(C₁-C₆)-alkylgruppe, eine (C₂-C₆)-Hydroxyalkylgruppe, eine (C₂-C₆)-Polyhydroxyalkylgruppe oder eine Gruppe R^{I}R^{II}N-CO-, worin R^{I} und R^{II} stehen unabhängig voneinander für ein Wasserstoffatom, eine (C₁-C₆)-Alkylgruppe, eine (C₁-C₆)-Alkenylgruppe, eine Arylgruppe oder eine Aryl-C₁-C₆-alkylgruppe, wobei R^{I} und R^{II} gemeinsam mit dem Stickstoffatom einen 5-, 6- oder 7-gliedrigen Ring bilden können, und
• R² steht für eine (C₁-C₆)-Alkylgruppe, eine (C₂-C₆)-Alkenylgruppe, eine gegebenenfalls substituierte Arylgruppe, eine Aryl-(C₁-C₆)-alkylgruppe, eine (C₁-C₆)-Hydroxyalkylgruppe, eine (C₂-C₆)-Polyhydroxyalkylgruppe, oder eine Gruppe R^{III}R^{IV}N-(CH₂)ₙ-, worin R^{III} und R^{IV} stehen unabhängig vonelnander für ein Wasserstoffatom, eine (C₁-C₆)-Alkylgruppe, eine (C₁-C₆)-Alkenylgruppe oder eine Aryl-C₁-C₆-alkylgruppe, wobei R^{III} und R^{IV} gemelnsam mit dem Stickstoffatom einen 5-, 6- oder 7-glledrigen Ring bilden können und n steht für eine Zahl 2, 3, 4, 5 oder 6,
• mindestens ein Rest aus R³ oder R⁵ steht für eine Methylgruppe und der andere Rest steht für ein Wasserstoffatom, eine (C₁-C₆)-Alkylgruppe, eine (C₂-C₆)-Alkenylgruppe, eine Arylgruppe, eine Aryl-(C₁-C₆)-alkylgruppe, eine (C₂-C₆)-Hydroxyalkylgruppe oder eine (C₂-C₆)-Polyhydroxyalkylgruppe,
• R⁴ steht für ein Wasserstoffatom, eine (C₁-C₆)-Alkylgruppe, eine (C₂-C₆)-Alkenylgruppe, eine (C₁-C₆)-Hydroxyalkylgruppe, eine (C₂-C₆)-Polyhydroxyalkylgruppe oder eine gegebenenfalls substituierte Arylgruppe,
• X⁻ ein physiologisch verträgliches Anion bedeutet
und als Komponente B mindestens eine reaktive Carbonylverbindung,

2. Mittel nach Anspruch 1, **dadurch gekennzeichnet, daß** der Rest R¹ für eine Carbamoylgruppe R^{I}R^{II}N-CO- steht, worin R^{I} und R^{II} unabhängig voneinander ein Wasserstoffatom oder eine (C₁-G₆)-Alkylgruppe, insbesondere eine Methylgruppe, bedeuten

3. Mittel nach einem der Ansprüche 1 oder 2, **dadurch gekennzeichnet, daß** die Verbindungen gemäß Formel I aus der Gruppe ausgewählt werden, bestehend aus Salzen mit physiologisch verträglichem Anion X⁻ des 1,2,4-Trimethyl-8-[(methylamino)carbonyl]imidazo[1,5-a]pyrimidiniums, des 1,2,3,4-Tetramethyl-8-[(methylamino)carbonyl]imidazo[1,5-a]pyrimidiniums, des 3-Ethyl-1,2,4-trimethyl-8-[(methylamino)carbonyl]imidazo[1,5-a]pyrimidiniums, sowie den Enaminformen der zuvor genannten Salze.

4. Mittel nach einem der Ansprüche 1 bis 3, **dadurch gekennzeichnet, daß** die Verbindungen der Komponente B ausgewählt wird aus Verbindungen gemäß Formel (Ca-1), 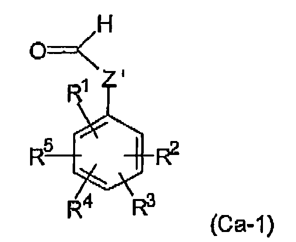 worin
• R¹, R² und R³ stehen unabhängig voneinander für ein Wasserstoffatom, ein Halogenatom, eine C₁-C₆-Alkylgruppe, eine Hydroxygruppe, eine C₁-C₆-Alkoxygruppe, eine C₁-C₆-Dialkylaminogruppe, eine Di(C₂-C₆-hydroxyalkyl)aminogruppe, eine Di(C₁-C₆-alkoxy-C₁-C₆₋alkyl)aminoguppe, eine C₁-C₈-Hydroxyalkyloxygruppe, eine Sulfonylgruppe, eine Carboxylgruppe, eine Sulfonsäuregruppe, eine Sulfonamidogruppe, eine Sulfonamidgruppe, eine Carbamoylgruppe, eine C₂-C₈-Acylgruppe, eine Acetylgruppe oder eine Nitrogruppe,
• Z¹ steht für eine direkte Bindung oder eine Vinylengruppe,
• R⁴ und R⁵ stehen für ein Wasserstoffatom oder bilden gemeinsam, zusammen mit dem Restmolekül einen 5- oder 6-gliederigen aromatischen oder aliphatischen Ring,

5. Mittel nach einem der Ansprüche 1 bis 4, **dadurch gekennzeichnet, daß** die Verbindungen der Komponente B ausgewählt werden, aus der Gruppe, bestehend aus 4-Hydroxy-3-methoxybenzaldehyd, 3,5-Dimethoxy-4-hydroxybenzaldehyd, 4-Hydroxy-1-naphthaldehyd, 4-Hydroxy-2-methoxybenzeldehyd, 3,4-Dihydroxy-5-methoxybenzaldehyd, 3,4,5-Trihydroxybenzaldehyd, 3,5-Dibrom-4-hydroxybenzaldehyd, 4-Hydroxy-3-nltrobenzaldehyd, 3-Brom-4-hydroxybenzaldehyd, 4-Hydroxy-3-methylbenzaldehyd, 3,5-Dimethyl-4-hydroxybenzaldehyd, 5-Brom-4-hydroxy-3-methoxybenzaldehyd, 4-Diethylamino-2-hydroxybenzaldehyd, 4-Dimethylamino-2-methoxybenzaldehyd, Coniferylaldehyd, 2-Methoxybenzaldehyd, 3-Methoxybenzaldehyd, 4-Methoxybenzaldehyd, 2-Ethoxybenzaldehyd, 3-Ethoxybenzaldehyd, 4-Ethoxybenzaldehyd, 4-Hydroxy-2,3-dimethoxy-benzaldehyd, 4-Hydroxy-2,5-dimethoxy-benzaldehyd, 4-Hydroxy-2,6-dimethoxy-benzaldehyd, 4-Hydroxy-2-methyl-benzaldehyd, 4-Hydroxy-2,3-dimethyl-benzaldehyd, 4-Hydroxy-2,5-dimethyl-benzaldehyd, 4-Hydroxy-2,6-dimethyl-benzaldehyd, 3,6-Diethoxy-4-hydroxy-benzaldehyd, 2,6-Dlethoxy-4-hydroxybenzaldehyd, 3-Hydroxy-4-methoxy-benzaldehyd, 2-Hydroxy-4-methoxybenzaldehyd, 2-Ethoxy-4-hydroxy-benzaldehyd, 3-Ethoxy-4-hydroxy-benzaldehyd, 4-Ethoxy-2-hydroxy-benzaldehyd, 4-Ethoxy-3-hydroxy-benzaldehyd, 2,3-Dimethoxybenzaldehyd, 2,4-Dimethoxybenzaldehyd, 2,5-Dimethoxybenzaldehyd, 2,6-Dimethoxybenzaldehyd, 3,4-Dimethoxybenzaldehyd, 3,5-Dimethoxybenzaldehyd, 2,3,4-Trimethoxybenzaldehyd, 2,3,5-Trimethoxybenzaldehyd, 2,3,6-Trimethoxybenzaldehyd, 2,4,6-Trimethoxybenzaldehyd, 2,4,5-Trimethoxybenzaldehyd, 2,5,6-Trimethoxybenzaldehyd, 2-Hydroxybenzaldehyd, 3-Hydroxybenzaldehyd, 4-Hydroxybenzaldehyd, 2,3-Dihydroxybenzaldehyd, 2,4-Dihydroxybenzaldehyd, 2,4-Dihydroxy-3-methyl-benzaldehyd, 2,4-Dihydroxy-5-methyl-benzaldehyd, 2,4-Dlhydroxy-6-methyl-benzaldehyd, 2,4-Dihydroxy-3-methoxy-benzaldehyd, 2,4-Dlhydroxy-5-methoxy-benzaldehyd, 2,4-Dihydroxy-6-methoxy-benzaldehyd, 2,5-Dlhydroxybenzaldehyd, 2,6-Dihydroxybenzaldehyd, 3,4-Dihydroxybenzaldehyd, 3,4-Dihydroxy-2-methyl-benzaldehyd, 3,4-Dihydroxy-5-methyl-benzaldehyd, 3,4-Dihydroxy-6-methyl-benzaldehyd, 3,4-Dihydroxy-2-methoxy-benzaldehyd, 3,5-Dihydroxybenzaldehyd, 2,3,4-Trihydroxybenzaldehyd, 2,3,6-Trihydroxybenzaldehyd, 2,3,6-Trihydroxybenzaldehyd, 2,4,6-Trihydroxybenzaldehyd, 2,4,5-Trihydroxybenzaldehyd, 2,5,6-Trihydroxybenzaldehyd, 4-Dimethylaminobenzaldehyd, 4-Diethylaminobenzaldehyd, 4-Dimethylamino-2-hydroxybenzaldehyd, 4-Pyrrolidinobenzaldehyd, 4-Morpholinobenzaldehyd, 2-Morpholinobenzaldehyd, 4-Piperidinobenzaldehyd, 3,5-Dichlor-4-hydroxybenzaldehyd, 4-Hydroxy-3,5-diiodbenzaldehyd, 3-Chlor-4-hydroxybenzaldehyd, 5-Chlor-3,4-dihydroxybenzaldehyd, 5-Brom-3,4-dihydroxybenzaldehyd, 3-Chlor-4-hydroxy-5-methoxybenzaldehyd, 4-Hydroxy-3-iod-5-methoxybenzaldehyd, 2-Methoxy-1-naphthaldehyd, 4-Methoxy-1-naphthaldehyd, 2-Hydroxy-1-naphthaldehyd, 2,4-Dihydroxy-1-napthaldehyd, 4-Hydroxy-3-methoxy-1-naphthaldehyd, 2-Hydroxy-4-methoxy-1-naphthaldehyd, 3-Hydroxy-4-methoxy-1-naphthaldehyd, 2,4-Dimethoxy-1-naphthaldehyd, 3,4-Dimethoxy-1-naphthaidehyd, 4-Dimethylamino-1-naphthaldehyd, 2-Nitrobenzaldehyd, 3-Nitrobenzaldehyd, 4-Nitrobenzaldehyd, 4-Methyl-3-nitrobenzaldehyd, 3-Hydroxy-4-nitrobenzaldehyd, 5-Hydroxy-2-nitrobenzaldehyd, 2-Hydroxy-5-nltrobenzaldehyd, 2-Hydroxy-3-nitrobenzaldehyd, 2-Fluor-3-nitrobenzaldehyd, 3-Methoxy-2-nitrobenzaldehyd, 4-Chlor-3-nitrobenzaldehyd, 2-Chlor-6-nitrobenzaldehyd, 5-Chlor-2-nitrobenzaldehyd, 4-Chlor-2-nitrobenzaldehyd, 2,4-Dinitrobenzaldehyd, 2,6-Dinitrobenzaldehyd, 2-Hydroxy-3-methoxy-5-nitrobenzaldehyd, 4,5-Dlmethoxy-2-nitrobenzaldehyd, 6-Nitroplperonal, 2-Nitropiperonal, 5-Nitrovanillin, 2,5-Dinitrosalicylaldehyd, 5-Brom-3-nitrosalicylaldehyd, 4-Nitro-1-naphthaldehyd, 2-Nitrozimtaldehyd, 3-Nitrozimtaldehyd, 4-Nitrozimtaldehyd, 4-Dimethylaminozimtaldehyd, 2-Dimethylaminobenzaldehyd, 2-Chlor-4-dimethylaminobenzaldehyd, 4-Dimethylamino-2-methylbenzaldehyd, 4-Diethylamino-zimtaldehyd, 4-Dibutylamino-benzaldehyd, 4-Diphenylamino-benzaldehyd, 4-(1-Imidazolyl)-benzaldehyd und Piperonal.

6. Mittel nach einem der Ansprüche 1 bis 5, **dadurch gekennzeichnet, daß** zusätzlich als Komponente C mindestens eine weitere CH-acide Verbindung enthalten ist, die von den Verbindungen gemäß Formel (I) verschieden ist.

7. Mittel nach Anspruch 6, **dadurch gekennzeichnet, daß** die CH-aciden Verbindungen der Komponente C ausgewählt werden, aus der Gruppe, bestehend aus mit physiologisch verträglichen Anionen gebildeten Salzen des 1,4-Dimethylchinoliniums, 1-Ethyl-4-methyl-chinoliniums, 1-Ethyl-2-methylchinofiniums, 1,2,3,3-Tetramethyl-3H-indoliums, 2,3-Dimethyl-benzothiazoliums, 2,3-Dimethyl-naphtho[1,2-d]thiazoliums, 3-Ethyl-2-methyl-naphtho[1,2-d]thiazoliums, 3-Ethyl-2-methyl-benzoxazoliums, 1,2,3-Trimethylchinoxaliniums, 3-Ethyl-2-methyl-benzothiazoliums, 1,2-Dihydro-1,3,4,6-tetramethyl-2-oxo-pyrimidiniums, 1,2-Dihydro-1,3,4-trimethyl-2-oxo-pyrimidiniums, 1,2-Dihydro-4,6-dimethyl-1,3-dipropyl-2-oxo-pyrimidiniums, 1,2-Dihydro-1,3,4,6-tetramethyl-2-thioxo-pyrimidiniums, 1,2-Dihydro-1,3,4,5,6-pentamethyl-2-oxo-pyrimidiniums, 2,5-Dimethyl-3-(2-propenyl)-1,3,4-thiadiazollums, 3-Ethyl-2,5-dimethyl-1,3,4-thiadiazoliums, 1,2-Dimethylchinoliniums und 1,3,3-Trimethyl-2-methylenindolin (Fischersche Base), Oxindol, 3-Methyl-1-phenyl-pyrazolin-5-on, Indan-1,2-dion, Indan-1,3-dion, Indan-1-on, 2-Amino-4-Imino-1,3-thiazolin-hydrochlorid, Benzoylacetonitril, 3-Dicyanmethylenindan-1-on,2-(2-Furanoyl)acetonitril, 2-(2-Theonyl)acetonitril, 2-(Cyanmethyl) benzimidazol, 2-(Cyanmethyl)-benzothiazol und 2-(2,6-Dimethyl-3-furanoyl)acetonitril.

8. Mittel nach einem der Ansprüche 1 bis 7, **dadurch gekennzeichnet, daß** die Verbindungen der Formel 1, die Verbindungen der Komponente B und gegebenenfalls die Verbindungen der Komponente C jeweils in einer Menge von 0,03 bis 65 mmol, insbesondere von 1 bis 40 mmol, bezogen auf 100 g des gesamten Färbemittels, enthalten sind.

9. Mittel nach einem der Ansprüche 1 bis 8, **dadurch gekennzeichnet, daß** es Farbverstärker ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Arginin, Histidin, Pyrrolidin, Prolin, Pyrrolidon, Pyrrolidon-5-carbonsäure, Pyrazol, 1,2,4-Triazol, Piperazidin oder deren beliebigen Gemischen enthält.

10. Mittel nach einem der Ansprüche 1 bis 9, **dadurch gekennzeichnet, daß** es zusätzlich als Oxidationsfarbstoffvorprodukt mindestens eine Entwicklerkomponente und gegebenenfalls mindestens eine Kupplerkomponente enthält,

11. Mittel nach einem der Ansprüche 1 bis 10, **dadurch gekennzeichnet, daß** es zusätzlich mindestens einen direktziehenden Farbstoff, vorzugsweise in einer Menge von 0,01 bis 20 Gew.-%, bezogen auf das gesamte Färbemittel, enthält.

12. Mittel nach einem der Ansprüche 1 bis 11, **dadurch gekennzeichnet, daß** es zusätzlich anionische, zwitterionische oder nichtionische Tenside enthält.

13. Verwendung von mindestens einer Verbindung gemäß Formel I gemäß Anspruch 1, in Kombination mit mindestens einer Verbindung der Komponente B gemäß Anspruch 1, als eine färbende Komponente in Haarfärbemitteln.

14. Verfahren zum Färben von keratinhaltigen Fasern, insbesondere menschlichen Haaren, worin ein Färbemittel, gemäß einem der Ansprüche 1 bis 12, sowie übliche kosmetische Inhaltsstoffe, auf die keratinhaltigen Fasern aufgebracht, einige Zeit, üblicherweise ca. 15-30 Minuten, auf der Faser belassen und anschließend wieder ausgespült oder mit einem Shampoo ausgewaschen wird.

15. Verfahren gemäß Anspruch 14, **dadurch gekennzeichnet, daß** in einem Zweischrlttverfahren die Komponente B gemäß Anspruch 1 vor oder nach Applikation der Verbindung gemäß Formel I gemäß Anspruch 1 auf die keratinhaltigen Fasern aufgebracht, die auf dem Haar erhaltene Mischung einige Zeit, üblicherwelse ca. 15-30 Minuten, auf der Faser belassen und anschließend wieder ausgespült oder mit einem Shampoo ausgewaschen wird.

16. Verfahren nach einem der Ansprüche 15 oder 16, **dadurch gekennzeichnet, daß** die keratinhaltigen Fasern, bevor ein Färbemittel gemäß einem der Ansprüche 1 bis 13 zur Anwendung kommt, im Rahmen einer Vorbehandlung mit einem Blondiermittel blondiert oder mit einem Oxidationsfärbemittel gefärbt wurden.

17. Verwendung von einer mindestens einer Verbindung gemäß Formel I gemäß Anspruch 1 zusammen mit mindestens einer reaktiven Carbonylverbindung als Komponente B zur Nuancierung von Oxidationsfärbungen von keratinhaltigen Fasern, insbesondere menschlichen Haaren,

18. Verwendung von mindestens einer Verbindung gemäß Formel I gemäß Anspruch 1 zusammen mit mindestens einer reaktiven Carbonylverbindung als Komponente B zur Farbauffrischung von mit oxidativen Färbemitteln gefärbten keratinhaltigen Fasern.

## Claims

1. Agent for coloring fibers containing keratin, especially human hair, comprising in a cosmetic carrier as component A at least one compound corresponding to Formula I and/or its enamine form , 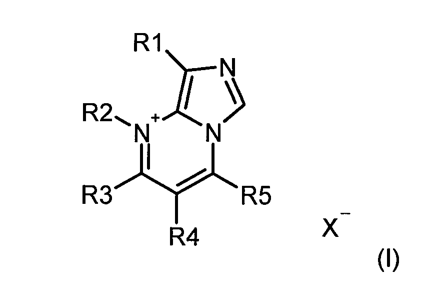 wherein
R¹ stands for a hydrogen atom, a halogen atom, carboxyl group, a (C₁-C₆) alkoxy group, (C₁-C₆) alkyl group, a (C₂-C₆) alkenyl group, an optionally substituted aryl group, an aryl (C₁-C₆) alkyl group, a (C₂-C₆) hydroxyalkyl group, a (C₂-C₆) polyhydroxyalkyl group or a R'R"N-CO- group, wherein R¹ and R^{II} stand independently of one another for a hydrogen atom, a (C₁-C₆) alkyl group, a (C₁-C₆) alkenyl group, an aryl group or an aryl C₁-C₆ alkyl group, wherein R^{I} and R^{II} together with the nitrogen atom can form a 5-, 6-or 7 membered ring, and
R² stands for a (C₁-C₆) alkyl group, a (C₂-C₆) alkenyl group, an optionally substituted aryl group, an aryl (C₁-C₆) alkyl group, a (C₁-C₆) hydroxyalkyl group, a (C₂-C₆) polyhydroxyalkyl group or a R^{III}R^{IV}N-(CH₂)ₙ- group, wherein R^{III} and R^{IV} stand independently of one another for a hydrogen atom, a (C₁-C₆) alkyl group, a (C₁-C₆) alkenyl group or an aryl C₁-C₆ alkyl group, wherein R^{III} and R^{IV} together with the nitrogen atom can form a 5-, 6- or 7 membered ring and n stands for a number 2, 3, 4, 5 or 6,
at least one R³ or R⁵ group stands for a methyl group and the other group stands for a hydrogen atom, a (C₁-C₆) alkyl group, a (C₂-C₆) alkenyl group, an aryl group, an aryl (C₁-C₆) alkyl group, a (C₂-C₆) hydroxyalkyl group or a (C₂-C₆) polyhydroxyalkyl group,
R⁴ stands for a hydrogen atom, a (C₁-C₆) alkyl group, a (C₂-C₆) alkenyl group, a (C₁-C₆) hydroxyalkyl group, a (C₂-C₆) polyhydroxyalkyl group or an optionally substituted aryl group,
X- means a physiologically compatible anion
and as component B at least one reactive carbonyl compound.

2. Agent according to claim 1, wherein the group R¹ stands for a carbamoyl group R^{I}R^{II}N-CO-, wherein R^{I} and R^{II} independently of one another mean a hydrogen atom or a (C₁-C₆) alkyl group, particularly a methyl group.

3. Agent according to one of claims 1 or 2, wherein the compounds corresponding to Formula I are selected from the group consisting of salts with physiologically compatible anions X- of 1,2,4-trimethyl-8-[(methylamino)carbonyl]imidazo[1,5-a]pyrimidinium, of 1,2,3,4-tetramethyl-8-[(methylamino)carbonyl]imidazo[1,5-a]pyrimidinium, of 3-ethyl-1,2,4-trimethyl-8-[(methylamino)carbonyl]imidazo[1,5-a]pyrimidinium, as well as of the enamine forms of the abovementioned salts.

4. Agent according to one of claims 1 to 3, wherein the compounds of component B are selected from compounds according to Formula (Ca-1), 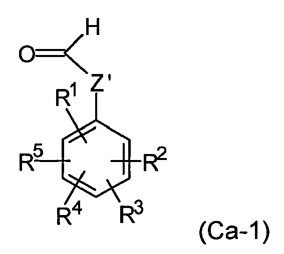 wherein
R¹, R² and R³ independently of each other stand for a hydrogen atom, a halogen atom, a C₁-C₆ alkyl group, a hydroxyl group, a C₁-C₆ alkoxy group, a C₁-C₆ dialkylamino group, a di(C₂-C₆ hydroxyalkyl)amino group, a di(C₁-C₆ alkoxy C₁-C₆ alkyl)amino group, a C₁-C₆ hydroxyalkyloxy group, a sulfonyl group, a carboxyl group, a sulfonic acid group, a sulfonamido group, a sulfonamide group, a carbamoyl group, a C₂-C₆ acyl group, an acetyl group or a nitro group,
Z' stands for a direct bond or a vinylene group,
R⁴ and R⁵ stand for a hydrogen atom or together form a 5-or 6-membered aromatic or aliphatic ring with the rest of the molecule.

5. Agent according to one of claims 1 to 4, wherein the compounds of component B are selected from the group consisting of 4-hydroxy-3-methoxybenzaldehyde, 3,5-dimethoxy-4-hydroxybenzaldehyde, 4-hydroxy-1-naphthaldehyde, 4-hydroxy-2-methoxybenzaldehyde, 3,4-dihydroxy-5-methoxybenzaldehyde, 3,4,5-trihydroxybenzaldehyde, 3,5-dibromo-4-hydroxybenzaldehyde, 4-hydroxy-3-nitrobenzaldehyde, 3-bromo-4-hydroxybenzaldehyde, 4-hydroxy-3-methylbenzaldehyde, 3,5-dimethyl-4-hydroxybenzaldehyde, 5-bromo-4-hydroxy-3-methoxybenzaldehyde, 4-diethylamino-2-hydroxybenzaldehyde, 4-dimethylamino-2-methoxybenzaldehyde, coniferyl aldehyde, 2-methoxybenzaldehyde, 3-methoxybenzaldehyde, 4-methoxybenzaldehyde, 2-ethoxybenzaldehyde, 3-ethoxybenzaldehyde, 4-ethoxybenzaldehyde, 4-hydroxy-2,3-dimethoxybenzaldehyde, 4-hydroxy-2,5-dimethoxybenzaldehyde, 4-hydroxy-2,6-dimethoxybenzaldehyde, 4-hydroxy-2-methylbenzaldehyde, 4-hydroxy-2,3-dimethylbenzaldehyde, 4-hydroxy-2,5-dimethylbenzaldehyde, 4-hydroxy-2,6-dimethylbenzaldehyde, 3,5-diethoxy-4-hydroxybenzaldehyde, 2,6-diethoxy-4-hydroxybenzaldehyde, 3-hydroxy-4-methoxybenzaldehyde, 2-hydroxy-4-methoxybenzaldehyde, 2-ethoxy-4-hydroxybenzaldehyde, 3-ethoxy-4-hydroxybenzaldehyde, 4-ethoxy-2-hydroxybenzaldehyde, 4-ethoxy-3-hydroxybenzaldehyde, 2,3-dimethoxybenzaldehyde, 2,4-dimethoxybenzaldehyde, 2,5-dimethoxybenzaldehyde, 2,6-dimethoxybenzaldehyde, 3,4-dimethoxybenzaldehyde, 3,5-dimethoxybenzaldehyde, 2,3,4-trimethoxybenzaldehyde, 2,3,5-trimethoxybenzaldehyde, 2,3,6-trimethoxybenzaldehyde, 2,4,6-trimethoxybenzaldehyde, 2,4,5-trimethoxybenzaldehyde, 2,5,6-trimethoxybenzaldehyde, 2-hydroxybenzaldehyde, 3-hydroxybenzaldehyde, 4-hydroxybenzaldehyde, 2,3-dihydroxybenzaldehyd, 2,4-dihydroxybenzaldehyde, 2,4-dihydroxy-3-methylbenzaldehyde, 2,4-dihydroxy-5-methylbenzaldehyde, 2,4-dihydroxy-6-methylbenzaldehyde, 2,4-dihydroxy-3-methoxybenzaldehyde, 2,4-dihydroxy-5-methoxybenzaldehyde, 2,4-dihydroxy-6-methoxybenzaldehyde, 2,5-dihydroxybenzaldehyde, 2,6-dihydroxybenzaldehyde, 3,4-dihydroxybenzaldehyde, 3,4-dihydroxy-2-methylbenzaldehyde, 3,4-dihydroxy-5-methylbenzaldehyde, 3,4-dihydroxy-6-methylbenzaldehyde, 3,4-dihydroxy-2-methoxybenzaldehyde, 3,5-dihydroxybenzaldehyde, 2,3,4-trihydroxybenzaldehyde, 2,3,5-trihydroxybenzaldehyde, 2,3,6-trihydroxybenzaldehyde, 2,4,6-trihydroxybenzaldehyde, 2,4,5-trihydroxybenzaldehyde, 2,5,6-trihydroxybenzaldehyde, 4-dimethylaminobenzaldehyde, 4-diethylaminobenzaldehyde, 4-dimethylamino-2-hydroxybenzaldehyde, 4-pyrrolidinobenzaldehyde, 4-morpholinobenzaldehyde, 2-morpholinobenzaldehyde, 4-piperidinobenzaldehyde, 3,5-dichloro-4-hydroxybenzaldehyde, 4-hydroxy-3,5-diiodobenzaldehyde, 3-chloro-4-hydroxybenzaldehyde, 5-chloro-3,4-dihydroxybenzaldehyde, 5-bromo-3,4-dihydroxybenzaldehyde, 3-chloro-4-hydroxy-5-methoxybenzaldehyde, 4-hydroxy-3-iodo-5-methoxybenzaldehyde, 2-methoxy-1-naphthaldehyde, 4-methoxy-1-naphthaldehyde, 2-hydroxy-1-naphthaldehyde, 2,4-dihydroxy-1-napthaldehyde, 4-hydroxy-3-methoxy-1-naphthaldehyde, 2-hydroxy-4-methoxy-1-naphthaldehyde, 3-hydroxy-4-methoxy-1-naphthaldehyde, 2,4-dimethoxy-1-naphthaldehyde, 3,4-dimethoxy-1-naphthaldehyde, 4-dimethylamino-1-naphthaldehyde, 2-nitrobenzaldehyde, 3-nitrobenzaldehyde, 4-nitrobenzaldehyde, 4-methyl-3-nitrobenzaldehyde, 3-hydroxy-4-nitrobenzaldehyde, 5-hydroxy-2-nitrobenzaldehyde, 2-hydroxy-5-nitrobenzaldehyde, 2-hydroxy-3-nitrobenzaldehyde, 2-fluoro-3-nitrobenzaldehyde, 3-methoxy-2-nitrobenzaldehyde, 4-chloro-3-nitrobenzaldehyde, 2-chloro-6-nitrobenzaldehyde, 5-chloro-2-nitrobenzaldehyde, 4-chloro-2-nitrobenzaldehyde, 2,4-dinitrobenzaldehyde, 2,6-dinitrobenzaldehyde, 2-hydroxy-3-methoxy-5-nitrobenzaldehyde, 4,5-dimethoxy-2-nitrobenzaldehyde, 6-nitropiperonal, 2-nitropiperonal, 5-nitrovanillin, 2,5-dinitrosalicylaldehyde, 5-bromo-3-nitrosalicylaldehyde, 4-nitro-1-naphthaldehyde, 2-nitrocinnamaldehyde, 3-nitrocinnamaldehyde, 4-nitrocinnamaldehyde, 4-dimethylaminocinnamaldehyde, 2-dimethylaminobenzaldehyde, 2-chloro-4-dimethylaminobenzaldehyde, 4-dimethylamino-2-methylbenzaldehyde, 4-diethylaminocinnamaldehyde, 4-dibutylaminobenzaldehyde, 4-diphenylaminobenzaldehyde, 4-(1-imidazolyl)benzaldehyde and piperonal.

6. Agent according to one of claims 1 to 5, wherein at least one further CH-acidic compound is additionally comprised as component C, which is different from the compounds according to Formula (I).

7. Agent according to claim 6, wherein the CH-acidic compounds of the component C are selected from the group consisting of salts formed with physiologically compatible anions with 1,4-dimethylquinolinium, 1-ethyl-4-methylquinolinium, 1-ethyl-2-methylquinolinium, 1,2,3,3-tetramethyl-3H-indolium, 2,3-dimethylbenzothiazolium, 2,3-dimethyl-naphtho[1,2-d]thiazolium, 3-ethyl-2-methyl-naphtho[1,2-d]thiazolium, 3-ethyl-2-methyl-benzoxazolium, 1,2,3-trimethylquinoxalinium, 3-ethyl-2-methylbenzothiazolium, 1,2-dihydro-1,3,4,6-tetramethyl-2-oxopyrimidinium, 1,2-dihydro-1,3,4-trimethyl-2-oxo-pyrimidinium, 1,2-dihydro-4,6-dimethyl-1,3-dipropyl-2-oxopyrimidinium, 1,2-dihydro-1,3,4,6-tetramethyl-2-thioxopyrimidinium, 1,2-dihydro-1,3,4,5,6-pentamethyl-2-oxopyrimidinium, 2,5-dimethyl-3-(2-propenyl)-1,3,4-thiadiazolium, 3-ethyl-2,5-dimethyl-1,3,4-thiadiazolium, 1,2-dimethylquinolinium and 1,3,3-trimethyl-2-methyleneindoline (Fischer base), oxindole, 3-methyl-1-phenyl-pyrazolin-5-one, indan-1,2-dione, indan-1,3-dione, indan-1-one, 2-amino-4-imino-1,3-thiazoline hydrochloride, benzoylacetonitrile, 3-dicyanomethyleneindan-1-one, 2-(2-furanoyl)acetonitrile, 2-(2-theonyl)acetonitrile, 2-(cyanomethyl)benzimidazole, 2-(cyanomethyl)benzothiazole and 2-(2,5-dimethyl-3-furanoyl)acetonitrile.

8. Agent according to one of the claims 1 to 7, wherein the compounds of Formula I, the compounds of the component B, and optionally the compounds of the component C are each comprised in an amount of 0.03 to 65 mmol, in particular from 1 to 40 mmol, based on 100 g of the total coloring agent.

9. Agent according to one of the claims 1 to 8, comprising color reinforcers selected from the group consisting of piperidine, piperidine-2-carboxylic acid, piperidine-3-carboxylic acid, piperidine-4-carboxylic acid, pyridine, 2-hydroxypyridine, 3-hydroxypyridine, 4-hydroxypyridine, imidazole, 1-methylimidazole, arginine, histidine, pyrrolidine, proline, pyrrolidone, pyrrolidone-5-carboxylic acid, pyrazole, 1,2,4-triazole, piperazidine or any mixtures thereof.

10. Agent according to one of claims 1 to 9, additionally comprising at least one developer component as the oxidation dye precursor and optionally at least one coupler component.

11. Agent according to one of claims 1 to 10, additionally comprising at least one substantive dye, preferably in a quantity of 0.01 to 20 wt.%, based on the total coloring agent.

12. Agent according to one of claims 1 to 11, additionally comprising anionic, zwitterionic or non-ionic surfactants.

13. Use of at least one compound according to Formula I according to claim 1, in combination with at least one compound of component B according to claim 1, as a coloration component in hair dyes.

14. Method for coloring keratin-containing fibers, especially human hair, wherein a coloring agent, according to one of claims 1 to 12, together with conventional cosmetic ingredients, are applied onto the keratin-containing fibers, left on the fibers for some time, usually ca. 15-30 minutes and then rinsed out again or washed out with a shampoo.

15. Method according to claim 14, wherein in a two-step method, the component B according to claim 1 is applied onto the keratin-containing fibers prior to or after the application of the compound according to Formula I according to claim 1, the resulting mixture on the hair is left on the fibers for some time, usually ca. 15-30 minutes, and then rinsed out again or washed out with a shampoo.

16. Method according to one of claims 14 or 15, wherein the keratin-containing fibers, in the context of a pre-treatment with a hair bleaching agent, were bleached or colored with an oxidation coloring agent before the application of a coloring agent according to one of claims 1 to 13.

17. Use of at least one compound according to Formula I according to claim 1 together with at least one reactive carbonyl compound as component B for nuancing oxidation colorations of keratin-containing fibers, especially human hair.

18. Use of at least one compound according to Formula I according to claim 1 together with at least one reactive carbonyl compound as component B for color refreshing keratin-containing fibers colored with oxidative coloring agents.

## Revendications

1. Agent pour la teinture de fibres kératiniques, en particulier des cheveux humains, contenant, dans un support cosmétique, à titre de composant A, au moins un composé répondant à la formule I et/ou sa forme énamine 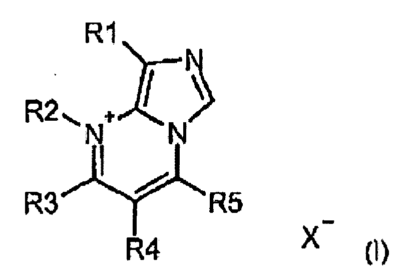 dans laquelle
• R¹ représente un atome d'hydrogène, un atome d'halogène, un groupe carboxyle, un groupe alcoxy en C₁-C₆, un groupe alkyle en C₁-C₆, un groupe alcényle en C₂-C₆, un groupe aryle le cas échéant substitué, un groupe arylalkyle en C₁-C₆, un groupe hydroxyalkyle en C₂-C₆, un groupe polyhydroxyalkyle en C₂-C₆, ou un groupe R^{I}R^{II}N-CO-, où R^{I} et R^{II} représentent, indépendamment l'un de l'autre, un atome d'hydrogène, un groupe alkyle en C₁-C₆, un groupe alcényle en C₁-C₆, un groupe aryle ou un groupe arylalkyle en C₁-C₆, R' et R" pouvant former ensemble avec l'atome d'azote un noyau à 5 membres, à 6 membres ou à 7 membres, et
• R² représente un groupe alkyle en C₁-C₆, un groupe alcényle en C₂-C₆, un groupe aryle le cas échéant substitué, un groupe arylalkyle en C₁-C₆, un groupe hydroxyalkyle en C₂-C₆, un groupe polyhydroxyalkyle en C₂-C₆, ou un groupe R^{III}R^{IV}N-(CH2)ₙ-, où R^{III} et R^{IV} représentent, indépendamment l'un de l'autre, un atome d'hydrogène, un groupe alkyle en C₁-C₆, un groupe alcényle en C₁-C₆ ou un groupe arylalkyle en C₁-C₆, R^{III} et R^{IV} pouvant former ensemble avec l'atome d'azote un noyau à 5 membres, à 6 membres ou à 7 membres et n représente un nombre égal à 2, 3, 4, 5 ou 6,
• au moins un des radicaux R³ et R⁵ représente un groupe méthyle, l'autre radical représentant un atome d'hydrogène, un groupe alkyle en C₁-C₆, un groupe alcényle en C₂-C₆, un groupe aryle, un groupe arylalkyle en C₁-C₆, un groupe hydroxyalkyle en C₂-C₆ ou un groupe polyhydroxyalkyle en C₂-C₆,
• R⁴ représente un atome d'hydrogène, un groupe alkyle en C₁-C₆, un groupe alcényle en C₂-C₆, un groupe hydroxyalkyle en C₁-_{C6}, un groupe polyhydroxyalkyle en C₂-C₆, ou un groupe aryle le cas échéant substitué,
• X⁻ représente un anion physiologiquement acceptable,
et, à titre de composant B, au moins un composé carbonyle réactif.

2. Agent selon la revendication 1, **caractérisé en ce que** le radical R¹ représente un groupe carbamoyle R^{I}R^{II}N-CO-, où R^{I} et R^{II} représentent, indépendamment l'un de l'autre, un atome d'hydrogène ou un groupe alkyle en C₁-C₆, en particulier un groupe méthyle.

3. Agent selon l'une quelconque des revendications 1 ou 2, **caractérisé en ce que** les composés répondant à la formule I sont choisis parmi le groupe constitué par des sels avec un anion physiologiquement acceptable X- du 1,2,4-trirnéthyl-8-[(méthylamino)carbonyl]imidazo[1,5-a]-pyrimidinium, du 1,2,3,4-tétraméthyl-8-[(méthylamino)carbonyl]-imidazo-[1,5-a]pyrimidinium, du 3-éthyl-1,2,4-triméthyl-8-[(méthylamino)-carbonyl]imidazo[1,5-a]pyrimidinium, ainsi que les formes énamines des sels mentionnés ci-dessus.

4. Agent selon l'une quelconque des revendications 1 à 3, **caractérisé en ce que** les composés du composant B sont choisis parmi des composés répondant à la formule (Ca-1), 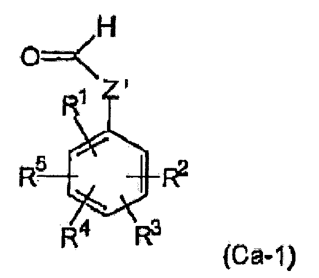 dans laquelle
• R¹, R² et R³ représentent, indépendamment l'un de l'autre, un atome d'hydrogène, un atome d'halogène, un groupe alkyle en C₁-C₆, un groupe hydroxyle, un groupe alcoxy en C₁-C₆, un groupe dialkyl(C₁-C₆)amino, un groupe dihydroxyalkyl(en C₂-C₆)amino, un groupe dialcoxy(en C₁-C₆)alkyl(en C₁-C₆)amino, un groupe hydroxyalkyl(en C₁-C₆)oxy, un groupe sulfonyle, un groupe carboxyle, un groupe d'acide sulfonique, un groupe sulfonamido, un groupe sulfonamide, un groupe carbamoyle, un groupe acyle en C₂-C₆, un groupe acétyle ou un groupe nitro ;
• Z' représente une liaison directe ou un groupe vinylène ;
• R⁴ et R⁵ représentent un atome d'hydrogène ou bien forment ensemble, conjointement avec la molécule résiduelle, un noyau aromatique ou aliphatique à 5 membres ou à 6 membres.

5. Agent selon l'une quelconque des revendications 1 à 4, **caractérisé en ce que** les composés du composant B sont choisis parmi le groupe constitué par le 4-hydroxy-3-méthoxybenzaldéhyde, le 3,5-diméthoxy-4-hydroxybenzaldéhyde, le 4-hydroxy-1-naphtaldéhyde, le 4-hydroxy-2-méthoxybenzaldéhyde, le 3,4-dihydroxy-5-méthoxybenzaldéhyde, le 3,4,5-trihydroxybenzaldéhyde, le 3,5-dibromo-4-hydroxybenzaldéhyde, le 4-hydroxy-3-nitrobenzaldéhyde, le 3-bromo-4-hydroxybenzaldéhyde, le 4-hydroxy-3-méthylbenzaldéhyde, le 3,5-diméthyl-4-hydroxybenzaldéhyde, le 5-bromo-4-hydroxy-3-méthoxybenzaldéhyde, le 4-diéthylamino-2-hydroxybenzaldéhyde, le 4-diméthylamino-2-méthoxybenzaldéhyde, le coniférylaldéhyde, le 2-méthoxybenzaldéhyde, le 3-méthoxybenzaldéhyde, le 4-méthoxybenzaldéhyde, le 2-éthoxybenzaldéhyde, le 3-éthoxybenzaldéhyde, le 4-éthoxybenzaldéhyde, le 4-hydroxy-2,3-diméthoxy-benzaldéhyde, le 4-hydroxy-2,5-diméthoxy-benzaldéhyde, le 4-hydroxy-2,6-diméthoxybenzaldéhyde, le 4-hydroxy-2-méthyl-benzaldéhyde, le 4-hydroxy-2,3-diméthyl-benzaldéhyde, le 4-hydroxy-2,5-diméthyl-benzaldéhyde, le 4-hydroxy-2,6-diméthyl-benzaldéhyde, le 3,5-diéthoxy-4-hydroxybenzaldéhyde, le 2,6-dléthoxy-4-hydroxy-benzaldéhyde, le 3-hydroxy-4-méthoxy-benzaldéhyde, le 2-hydroxy-4-méthoxy-benzaldéhyde, le 2-éthoxy-4-hydroxy-benzaldéhyde, le 3-éthoxy-4-hydroxy-benzaldéhyde, le 4-éthoxy-2-hydroxy-benzaldéhyde, le 4-éthoxy-3-hydroxy-benzaldéhyde, le 2,3-diméthoxybenzaldéhyde, le 2,4-diméthoxybenzaldéhyde, le 2,5-dlméthoxybenzaldéhyde, le 2,6-diméthoxybenzaldéhyde, le 3,4-diméthoxybenzaldéhyde, le 3,5-diméthoxyhenzaldéhyde, le 2,3,4-triméthoxybenzaldéhyde, le 2,3,5-triméthoxybenzaldéhyde, le 2,3,6-triméthoxybenzaldéhyde, le 2,4,6-triméthoxybenzaldéhyde, le 2,4,5-triméthoxybenzaldéhyde, le 2,5,6-triméthoxybenzaldéhyde, le 2-hydroxybenzaldéhyde, le 3-hydroxybenzaldéhyde, le 4-hydroxybenzaldéhyde, le 2,3-dihydroxybenzaldéhyde, le 2,4-dihydroxybenzaldéhyde, le 2,4-dihydroxy-3-méthyl-benzaldéhyde, le 2,4-dihydroxy-5-méthyl-benzaldéhyde, le 2,4-dihydroxy-6-méthyl-benzaldéhyde, le 2,4-dihydroxy-3-méthoxy-benzaldéhyde, le 2,4-dihydroxy-5-méthoxy-benzaldéhyde, le 2,4-dihydroxy-6-méthoxybenzaldéhyde, le 2,5-dihydroxyhenzaldéhyde, le 2,6-dihydroxybenzaldéhyde, le 3,4-dihydroxybenzaldéhyde, le 3,4-dihydroxy-2-méthyl-benzaldéhyde, le 3,4-dihydroxy-5-méthyl-benzaldéhyde, le 3,4-dihydroxy-6-méthyl-benzaldéhyde, le 3,4-dihydroxy-2-méthoxybenzaldéhyde, le 3,5-dihydroxybenzaldéhyde, le 2,3,4-trihydroxybenzaldéhyde, le 2,3,5-trihydroxybenzaldéhyde, le 2,3,6-trihydroxybenzaldéhyde, le 2,4,6-trihydroxybenzaldéhyde, le 2,4,5-trihydroxybenzaldéhyde, le 2,5,6-trihydroxybenzaldéhyde, le 4-diméthylaminobenzaldéhyde, le 4-diéthylaminobenzaldéhyde, le 4-diméthylamino-2-hydroxybenzaldéhyde, le 4-pyrrolidinobenzaldéhyde, le 4-morpholinobenzaldéhyde, le 2-morpholinobenzaldéhyde, le 4-pipéridinobenzaldéhyde, le 3,5-dichloro-4-hydroxybenzaldéhyde, le 4-hydroxy-3,5-diiodo-benzaldéhyde, le 3-chloro-4-hydroxybenzaldéhyde, le 5-chloro-3,4-dihydroxybenzaldéhyde, le 5-bromo-3,4-dihydroxybenzaldéhyde, le 3-chloro-4-hydroxy-5-méthoxybenzaldéhyde, le 4-hydroxy-3-iodo-5-méthoxybenzaldéhyde, le 2-méthoxy-1-naphtaldéhyde, le 4-méthoxy-1-naphtaldéhyde, le 2-hydroxy-1-naphtaldéhyde, le 2,4-dihydroxy-1-naptaldéhyde, le 4-hydroxy-3-méthoxy-1-naphtaldéhyde, le 2-hydroxy-4-méthoxy-1-naphtaldéhyde, le 3-hydroxy-4-méthoxy-1-naphtaldéhyde, le 2,4-diméthoxy-1-naphtaldéhyde, le 3,4-diméthoxy-1-naphtaldéhyde, le 4-diméthylamino-1-naphtaldéhyde, le 2-nitrobenzaldéhyde, le 3-nitrobenzaldéhyde, le 4-nitrobenzaldéhyde, le 4-méthyl-3-nitrobenzaldéhyde, le 3-hydroxy-4-nitrobenzaldéhyde, le 5-hydroxy-2-nitrobenzaldéhyde, le 2-hydroxy-5-nitrobenzaldéhyde, le 2-hydroxy-3-nitrobenzaldéhyde, le 2-fluoro-3-nitrobenzaldéhyde, le 3-méthoxy-2-nitrobenzaldéhyde, le 4-chloro-3-nitrobenzaldéhyde, le 2-chloro-6-nitrobenzaldéhyde, le 5-chloro-2-nitrobenzaldéhyde, le 4-chloro-2-nitrobenzaldéhyde, le 2,4-dinitrobenzaldéhyde, le 2,6-dinitrobenzaldéhyde, le 2-hydroxy-3-méthoxy-5-nitrobenzaldéhyde, le 4,5-dlméthoxy-2-nitrobenzaldéhyde, le 6-nitropipéronal, le 2-nitropipéronal, la 5-nitrovanilline, le 2,5-dinitrosalicylaldéhyde, le 5-bromo-3-nitrosalicylaldéhyde, le 4-nitro-1-5-naphtaldéhyde, le 2-nitrocinnamaldéhyde, le 3-nitrocinnamaldéhyde, le 4-nitrocinnamaldéhyde, le 4-diméthylaminocinnamaldéhyde, le 2-diméthylaminobenzaldéhyde, le 2-chloro-4-diméthylamino-benzaldéhyde, le 4-diméthylamino-2-méthylbenzaldéhyde, le 4-diéthylamino-cinnamaldéhyde, le 4-dibutylamino-benzaldéhyde, le 4-diphénylamino-benzaldéhyde, le 4-(1-imidazolyl)-benzaldéhyde et le pipéronal.

6. Agent selon l'une quelconque des revendications 1 à 5, **caractérisé en ce qu'**il contient en outre, à titre de composant C, un autre composé à liaison C-H acide, qui est différent des composés répondant à la formule (I).

7. Agent selon la revendication 6, **caractérisé en ce que** les composés à liaison C-H-acide sont choisis parmi le groupe constitué par des sels formés avec des anions physiologiquement acceptables, du 1,4-diméthylquinolinium, du 1-éthyl-4-méthyl-quinolinlum, du 1-éthyl-2-méthylquinolinium, du 1,2,3,3-tétraméthyl-3H-indolium, du 2,3-diméthyl-benzothiazolium, du 2,3-diméthyl-naphto[1,2-d]thiazolium, du 3-éthyl-2-méthyl-naphto[1,2-d]thiazolium, du 3-éthyl-2-méthyl-benzoxazolium, du 1,2,3-triméthylquinoxalinium, du 3-éthyl-2-méthyl-benzothiazolium, du 1,2-dihydro-1,3,4,6-tétraméthyl-2-oxo-pyrimidinium, du 1,2-dihydro-1,3,4-triméthyl-2-oxo-pyrimidinium, du 1,2-dihydro-4,6-diméthyl-1,3-dipropyl-2-oxo-pyrimidinium, du 1,2-dihydro-1,3,4,6-tétraméthyl-2-thioxo-pyrimidinium, du 1,2-dihydro-1,3,4,5,6-pentarnéthyl-2-oxo-pyrimidinium, du 2,5-diméthyl-3-(2-propényl)-1,3,4-thiadiazolium, du 3-éthyl-2,5-diméthyl-1,3,4-thiadiazolium, du 1,2-diméthylquinolinium et de la 1,3,3-triméthyl-2-méthylène-indoline (base de Fischer), l'oxindole, la 3-méthyl-1-phényl-pyrazolin-5-one, l'indan-1,2-dione, l'indan-1,3-dione, l'indan-1-one, le chlorhydrate de 2-amino-4-imino-1,3-thiazoline, le benzoylacétonitrile, la 3-dicyanométhylène-indan-1-one, le 2-(2-furanoyl)acétonitrile, le 2-(2-théonyl)acétonitrile, le 2-(cyanométhyl)benzimidazole, le 2-(cyanométhyl)benzothiazole et le 2-(2,5-diméthyl-3-furanoyl)acétonitrile.

8. Agent selon l'une quelconque des revendications 1 à 7, **caractérisé en ce qu'**il contient les composés répondant à la formule I, les composés du composant B et le cas échéant les composés du composant C, respectivement en une quantité de 0,03 à 65 millimoles, en particulier de 1 à 40 millimoles, rapportés à 100 g de la teinture totale.

9. Agent selon l'une quelconque des revendications 1 à 8, **caractérisé en ce qu'**il contient un renforçateur des couleurs choisi parmi le groupe constitué par la pipéridine, l'acide pipéridine-2-carboxylique, l'acide pipéridine-3-carboxylique, l'acide pipéridine-4-carboxylique, la pyridine, la 2-hydroxypyridine, la 3-hydroxypyridine, la 4-hydroxypyridine, l'imidazole, le 1-méthylimidazole, l'arginine, l'histidine, la pyrrolidine, la proline, la pyrrolidone, l'acide pyrrolidone-5-carboxylique, le pyrazole, le 1,2,4-triazole, la pipérazidine ou l'un quelconque de leurs mélanges.

10. Agent selon l'une quelconque des revendications 1 à 9, **caractérisé en ce qu'**il contient en outre, à titre de précurseur de colorants d'oxydation, au moins un composant faisant office de développeur et le cas échéant au moins un composant faisant office de coupleur.

11. Agent selon l'une quelconque des revendications 1 à 10, **caractérisé en ce qu'**il contient en outre au moins un colorant montant directement sur la fibre, de préférence en une quantité de 0,01 à 20 % en poids, rapportés à la teinture totale.

12. Agent selon l'une quelconque des revendications 1 à 11, **caractérisé en ce qu'**il contient en outre des agents tensioactifs anioniques, zwitterioniques ou non ioniques.

13. Utilisation d'au moins un composé répondant à la formule I selon la revendication 1, en combinaison avec au moins un composé du composant B selon la revendication 1, à titre d'un composant de coloration dans des teintures capillaires.

14. Procédé pour la teinture de fibres kératiniques, en particulier de cheveux humains, dans lequel on applique sur les fibres kératiniques, une teinture, selon l'une quelconque des revendications 1 à 12, ainsi que les constituants cosmétiques habituels, on laisse agir sur les fibres pendant un certain temps, habituellement pendant un laps de temps d'environ 15 à 30 minutes, et on l'élimine ensuite par rinçage ou bien par lavage avec un shampooing.

15. Procédé selon la revendication 14, **caractérisé en ce que**, dans un procédé en deux étapes, on applique sur les fibres kératiniques, le composant B. selon la revendication 1, avant ou après l'application du composé répondant à la formule I selon la revendication 1, on laisse agir sur les fibres le mélange obtenu sur les cheveux pendant un certain temps, habituellement pendant un laps de temps d'environ 15 à 30 minutes, et on l'élimine ensuite par rinçage ou bien par lavage avec un shampooing.

16. Procédé selon l'une quelconque des revendications 14 ou 15, **caractérisé en ce qu'**on soumet les fibres kératiniques, avant d'utiliser une teinture selon l'une quelconque des revendications 1 à 13, dans le cadre d'un prétraitement, à une décoloration avec un agent de décoloration ou à une teinture avec un colorant d'oxydation.

17. Utilisation d'au moins un composé répondant à la formule I selon la revendication 1, de manière conjointe avec au moins un composé carbonyle réactif à titre de composant B pour le nuançage de teintures d'oxydation de fibres kératiniques, en particulier de cheveux humains.

18. Utilisation d'au moins un composé répondant à la formule I selon la revendication 1, de manière conjointe avec au moins un composé carbonyle réactif à titre de composant B pour le rafraîchissement des couleurs de fibres kératiniques teintes avec des colorants d'oxydation.
